# EUROPEAN PATENT APPLICATION

(11) **EP 2 264 064 A1**
(43) Date of publication of application: **22.12.2010**
(21) Application number: 10011852.0
(22) Date of filing: 28.04.2000
(51) Int. Cl.: C07K 14/575, A61K 47/48

(54) **Modified exendins and exendin agonists**

(30) Priority: 30.04.1999 US 132018 P
(62) Divisional of application: 00928685.7
(71) Applicant: Amylin Pharmaceuticals Inc., San Diego, CA 92121 (US)
(72) Inventor: Young, Andrew, Point Loma, CA 92166 (US); Prickett, Kathryn, San Diego, CA 92126 (US)
(74) Representative: Raynor, Stuart Andrew

(57) **Abstract**

Novel modified exendins and exendin agonists having an exendin or exendin agonist linked to one or more polyethylene glycol polymers, for example, and related formulations and dosages and methods of administration thereof are provided. These modified exendins and exendin agonists, compositions and methods are useful in treating diabetes and conditions that would be benefited by lowering plasma glucose or delaying and/or slowing gastric emptying or inhibiting food intake.

## Description

### RELATED APPLICATIONS

This application claims priority to, and the benefit of, United States provisional patent application serial no. 60/132,018, filed April 30, 1999, which application is hereby incorporated by reference in its entirity.

### FIELD OF THE INVENTION

The present invention relates to novel modified exendins and exendin agonists having an exendin or exendin agonist peptide linked to one or more polyethylene glycol polymers (or other molecular weight increasing agents), and related products and methods that are useful, for example, in the treatment of diabetes, including Type 1 and 2 diabetes, in the treatment of disorders which would be benefited by agents which modulate plasma glucose levels, and in the treatment of disorders which would be benefited by the administration of agents useful in modulating glucagon or triglyceride levels, or the rate of gastric emptying or food intake, including obesity, eating disorders, and insulin-resistance syndrome.

### BACKGROUND

The following description includes information that may be useful in understanding the present invention. It is not an admission that any of the information provided herein is prior art to the presently claimed invention, nor that any of the publications specifically or implicitly referenced are prior art to that invention.

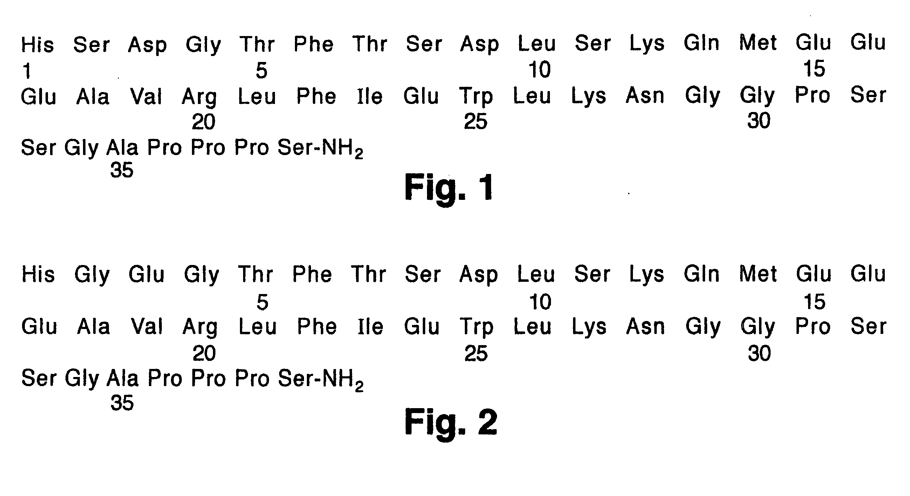

The exendins are peptides that are found in the salivary secretions of the Gila monster and the Mexican Bearded Lizard, reptiles that are endogenous to Arizona and Northern Mexico. Exendin-3 [SEQ. ID. NO. 1] is present in the salivary secretions of *Heloderma horridum* (Mexican Beaded Lizard), and exendin-4 [SEQ. ID. NO. 2] is present in the salivary secretions of *Heloderm suspectum* (Gila monster) (Eng, J., et al., J. Biol. Chem., 265:20259-62, 1990; Eng, J., et al., J. Biol. Chem., 267:7402-05, 1992). The amino acid sequence of exendin-3 is shown in Figure 1. The amino acid sequence of exendin-4 is shown in Figure 2. Exendin-4 was first thought to be a (potentially toxic) component of the venom. It now appears that exendin-4 is devoid of toxicity, and that it instead is made in salivary glands in the Gila monster.

The exendins have some sequence similarity to several members of the glucagon-like peptide family, with the highest homology, 53%, being to GLP-1[7-36]NH₂ [SEQ. ID. NO. 3] (Goke, et al., J. Biol. Chem., 268:19650-55, 1993). GLP-1[7-36]NH₂, also sometimes referred to as proglucagon[78-107] or simply "GLP-1", has an insulinotropic effect, stimulating insulin secretion from pancreatic beta-cells; GLP-1 has also been reported to inhibit glucagon secretion from pancreatic alpha-cells (Ørsov, et al., Diabetes, 42:658-61, 1993; D'Alessio, et al., J. Clin. Invest., 97:133-38, 1996). GLP-1 has been reported to inhibit gastric emptying (Willms B, et al., J. Clin. Endocrinol. Metab. 81 (1): 327-32, 1996; Wettergren A, et al., Dig. Dis. Sci . 38 (4): 665-73, 1993), and gastric acid secretion (Schjoldager BT, et al., Dig. Dis. Sci. 34 (5): 703-8, 1989; O'Halloran DJ, et al., J. Endocrinol. 126 (1): 169-73, 1990; Wettergren A, et al., Dig. Dis. Sci. 38 (4): 665-73, 1993)). GLP-1[7-37], which has an additional glycine residue at its carboxy terminus, is reported to stimulate insulin secretion in humans (Ørsov, et al., Diabetes, 42:658-61, 1993). Other reports relate to the inhibition of glucagon secretion (Creutzfeldt WOC, et al., Glucagonostatic actions and reduction of fasting hyperglycemia by exogenous glucagon-like peptide I(7-36) amide in Type 1 diabetic patients, Diabetes Care 1996;19(6):580-6), and a purported role in appetite control (Turton MD, et al., A role for glucagon-like peptide-1 in the central regulation of feeding, Nature 1996 Jan;379(6560):69-72).A transmembrane G-protein adenylate-cyclase-coupled receptor, said to be responsible at least in part for the insulinotropic effect of GLP-1, has reportedly been cloned from a beta-cell line (Thorens, Proc. Natl. Acad. Sci. USA 89:8641-45, 1992). GLP-1 has been the focus of significant investigation in recent years due to its reported action on the amplification of stimulated insulin production (Byrne MM, Goke B. Lessons from human studies with glucagon-like peptide-1: Potential of the gut hormone for clinical use. In: Fehmann HC, Goke B. Insulinotropic Gut Hormone Glucagon-Like Peptide 1. Basel, Switzerland: Karger, 1997:219-33).

GLP-1 has also been reported to restore islet glucose sensitivity in aging rats, restoring their glucose tolerance to that of younger rats (Egan JM, et al., Diabetologia 1997 Jun;40(Suppl 1):A130). However, the short duration of biological action of GLP-1 *in vivo* is one feature of the peptide that has hampered its development as a therapeutic agent. Various methods have been tried to prolong the half-life of GLP-1 or GLP-1(7-37), including attempts to alter their amino acid sequences and to deliver them using certain formulations (see, e.g., European Patent Application, entitled "Prolonged Delivery of Peptides," by Darley, et al., publication number 0 619 322 A2, regarding the inclusion of polyethylene glycol in formulations containing GLP-1 (7-37)).

Pharmacological studies have led to reports that exendin-4 can act at GLP-1 receptors in vitro on certain insulin-secreting cells, at dispersed acinar cells from guinea pig pancreas, and at parietal cells from stomach; the peptide is also reported to stimulate somatostatin release and inhibit gastrin release in isolated stomachs (Goke, et al., J. Biol. Chem. 268:19650-55, 1993; Schepp, et al., Eur. J. Pharmacol., 69:183-91, 1994; Eissele, et al., Life Sci., 55:629-34, 1994). Exendin-3 and exendin-4 were reportedly found to stimulate cAMP production in, and amylase release from, pancreatic acinar cells (Malhotra, R., et al., Regulatory Peptides, 41:149-56, 1992; Raufman, et al., J. Biol. Chem. 267:21432-37, 1992; Singh, et al., Regul. Pept. 53:47-59, 1994). Exendin-4 has a significantly longer duration of action than GLP-1. For example, in one experiment, glucose lowering by exendin-4 in diabetic mice was reported to persist for several hours, and, depending on dose, for up to 24 hours (Eng, J. Prolonged effect of exendin-4 on hyperglycemia of db/db mice, Diabetes 1996 May; 45(Suppl 2):152A (abstract 554)). Based on their insulinotropic activities, the use of exendin-3 and exendin-4 for the treatment of diabetes mellitus and the prevention of hyperglycemia has been proposed (Eng, U.S. Patent No. 5,424,286).

The results of an investigation which showed that exendins are not the species homolog of mammalian GLP-1 was reported by Chen and Drucker who cloned the exendin gene from the Gila monster (J. Biol. Chem. 272(7):4108-15 (1997)). The observation that the Gila monster also has separate genes for proglucagons (from which GLP-1 is processed), that are more similar to mammalian proglucagon than exendin, indicated that exendins are not merely species homologs of GLP-1.

Methods for regulating gastrointestinal motility using exendin agonists are described in commonly owned U.S. Patent Application Serial No. 08/908,867, filed August 8, 1997 entitled "Methods for Regulating Gastrointestinal Motility," which application is a continuation-in-part of U.S. Patent Application Serial No. 08/694,954, filed August 8, 1996.

Methods for reducing food intake using exendin agonists are described in commonly owned U.S. Patent Application Serial No. 09/003,869, filed January 7, 1998, entitled "Use of Exendin and Agonists Thereof for the Reduction of Food Intake," which claims the benefit of U.S. Provisional Application Nos. 60/034,905 filed January 7, 1997, 60/055,404 filed August 7, 1997, 60/065,442 filed November 14, 1997 and 60/066,029 filed November 14, 1997.

Novel exendin agonist compounds are described in commonly owned PCT Application Serial No. PCT/US98/16387 filed August 6, 1998, entitled "Novel Exendin Agonist Compounds," which claims the benefit of U.S. Patent Application Serial No. 60/055,404, filed August 8, 1997.

Other novel exendin agonists are described in commonly owned PCT Application Serial No. PCT/US98/24210, filed November 13, 1998, entitled "Novel Exendin Agonist Compounds," which claims the benefit of U.S. Provisional Application No. 60/065,442 filed November 14, 1997.

Still other novel exendin agonists are described in commonly owned PCT Application Serial No. PCT/US98/24273, filed November 13, 1998, entitled "Novel Exendin Agonist Compounds," which claims the benefit of U.S. Provisional Application No. 60/066,029 filed November 14, 1997.

Other recent advances in exendin related technology are described in U.S. Provisional Patent Application Serial No. 60/075,122, filed February 13, 1998, entitled "Inotropic and Diuretic Effects of Exendin and GLP-1" and in U.S. Provisional Patent Application Serial No. 60/116,380, filed January 14, 1998, entitled "Novel Exendin Agonist Formulations and Methods of Administration Thereof".

Polyethylene glycol (PEG) modification of therapeutic peptides and proteins may yield both advantages and disadvantages. While PEG modification may lead to improved circulation time, reduced antigenicity and immunogenicity, improved solubility, resistance to proteolysis, improved bioavailability, reduced toxicity, improved stability, and easier formulation of peptides (See, Francis et al., International Journal of Hematology, 68:1-18, 1998) problems with PEGylation in most cases is substantial reduction in bioactivity. Id. In addition, most methods involve use of linkers that have several types of adverse effects including immunogenicity, instability, toxicity, and reactivity. *Id*.

Modified exendins and exendin agonists and related formulations, dosage formulations, and methods that solve these problems and that are useful in the delivery of therapeutically effective amounts of exendins and exendin agonists are described and claimed herein.

The contents of the above-identified articles, patents, and patent applications, and all other documents mentioned or cited herein, are hereby incorporated by reference in their entirety. The inventors reserve the right to physically incorporate into this application any and all materials and information from any such articles, patents, patent applications, or other documents mentioned or cited herein.

### SUMMARY OF THE INVENTION

The present invention relates to novel modified exendins and exendin agonists having an exendin or exendin agonist linked to one or more molecular weight increasing compounds, of which polyethylene glycol polymers (or other molecular weight increasing agents), and related products and methods. Such products and methods that are useful for many applications, including, for example, in the treatment of diabetes, including Type 1 and 2 diabetes, gestational diabetes (see U.S. patent application serial no. 09/323,867, entitled, "Use of Exendins and Agonists Thereof For The Treatment of Gestational Diabetes Mellitus," filed June 1, 1999), in the treatment of disorders which would be benefited by agents which modulate plasma glucose levels, in the treatment of disorders which would be benefited by the administration of agents useful in modulating the rate of gastric emptying or food intake, including obesity, eating disorders, and insulin-resistance syndrome, and to modulate triglyceride levels and to treat subjects suffering from dyslipidemia (i.e., increased LDL cholesterol, increased VLDL cholesterol, and/or decreased HDL cholesterol)(see U.S. provisonal patent application serial no. 60/175,365, entitled, "Use of Exendins and Agonists Thereof for Modulation of Triglyceride Levels and Treatment of Dyslipidemia," filed January 10, 2000). The methods are also useful for lowering plasma lipid levels, reducing cardiac risk, reducing the appetite, and reducing the weight of subjects. Still other embodiments concern methods for suppressing glucagon secretion (see U.S. provisonal patent application serial no. 60/132,017, entitled, "Methods for Glucagon Suppression," filed April 30, 1999, which is commonly owned). Pharmaceutical compositions for use in the methods of the invention are also disclosed.

The present invention is related to the surprising discovery that exendin is cleared from the plasma almost entirely by renal filtration, and not primarily by proteolytic degradation, as occurs for many other biologically active peptides, for example, GLP-1. This surprising discovery supports the determination that PEGylation or other modification of exendin or exendin agonists to increase molecular size, will have pharmaceutical benefit.

Thus, the present invention provides a modified exendin or exendin agonist having an exendin or exendin agonist linked to one or more polyethylene glycol polymers or other molecular weight increasing compounds. A "molecular weight increasing compound" is one that can be conjugated to an exendin or exendin agonist and thereby increase the molecular weight of the resulting conjugate. Representative examples of molecular weight increasing compounds, in addition to PEG, are polyamino acids (e.g., poly-lysine, poly-glutamic acid, and poly-aspartic acid; see Gombotz, et al. (1995), Bioconjugate Chem., vol. 6: 332-351; Hudecz, et al. (1992), Bioconjugate Chem., vol. 3, 49-57; Tsukada, et al. (1984), J. Natl. Cancer Inst., vol 73,: 721-729; Pratesi, et al. (1985), Br. J. Cancer, vol. 52: 841-848), particularly those of the L conformation, pharmacologically inactive proteins (e.g., albumin; see Gombotz, et al. (1995) and the references cited therein), gelatin (see Gombotz, et al. (1995) and the references cited therein), succinyl-gelatin (see Gombotz, et al. (1995) and the references cited therein), (hydroxypropyl)-methacrylamide (see Gombotz, et al. (1995) and the references cited therein), a fatty acid, a olysaccaride, a lipid amino acid, and dextran.

In preferred embodiments, the modified exendin or exendin agonist has a molecular weight that is greater than the molecular weight of the exendin or exendin agonist (preferably about 10%, 50% or 90% greater), the modified exendin or exendin agonist has a negative charge that is greater than the negative charge of the exendin or exendin agonist (preferably about 10%, 50% or 90% greater), the modified exendin or exendin agonist has a kidney clearance that is less than the kidney clearance of the exendin or exendin agonist (preferably about 10%, 50% or 90% less), the modified exendin or exendin agonist has a half-life that is greater than the half-life of the exendin or exendin agonist (preferably about 10%, 50% or 90% greater), the modified exendin or exendin agonist has a immunogenicity/antigenicity that is less than the immunogenicity/antigenicity of the exendin or exendin agonist, the modified exendin or exendin agonist has a solubility that is greater than the solubility of the exendin or exendin agonist (preferably about 10%, 50% or 90% greater), the modified exendin or exendin agonist has a proteolysis rate that is less than the proteolysis rate of the exendin or exendin agonist (preferably about 10%, 50% or 90% less), the modified exendin or exendin agonist has a toxicity that is less than the toxicity of the exendin or exendin agonist, the modified exendin or exendin agonist has a stability that is greater than the stability of the exendin or exendin agonist, and/or the modified exendin or exendin agonist has a permeability/biological function that is greater or less than the permeability/biological function of the exendin or exendin agonist (preferably about 10%, 50% or 90% greater or less).

The exendin or exendin agonist may be linked to one, two or three polyethylene glycol polymers or other molecular weight increasing agents. The polyethylene glycol polymers (or other molecular weight increasing agents) may preferably have molecular weights between 500 and 20,000. In a preferred embodiment, the modified exendin or exendin agonist is one of compounds 201-230, more preferably one of compounds 209, 210 and 213, or one of compounds 201 and 202, or one of compounds 216 and 217 (See Example 4 below).

The polyethylene glycol polymers (or other molecular weight increasing agents) are preferably linked to an amino, carboxyl, or thio group, and may be linked by N or C termini of side chains of lysine, aspartic acid, glutamic acid, or cysteine, or alternatively, the polyethylene glycol polymers or other molecular weight increasing agents may be linked with diamine and dicarboxylic groups. The exendin or exendin agonist is preferably linked to the polyethylene glycol polymers or other molecular weight increasing agents through an epsilon amino group on a lysine amino acid of the exendin or exendin agonist.

The present invention also features a method of making a modified exendin or exendin agonist. The method involves linking one or more polyethylene glycol polymers or other molecular weight increasing agents to an exendin or exendin agonist. In preferred embodiments, the linking is performed by solid-phase synthesis.

The present invention also provides a method of treating a disease benefited by administration of an exendin or exendin agonist. The method involves providing a modified exendin or exendin agonist of the invention to a patient having such a disease and thereby treating the disease. Exemplary diseases include postprandial dumping syndrome, postprandial hyperglycemia, impaired glucose tolerance, a condition or disorder which can be alleviated by reducing food intake, obesity, an eating disorder, insulin-resistance syndrome, diabetes mellitus, and a hyperglycemic condition. In a preferred embodiment, the postprandial hyperglycemia is a consequence of Type 2 diabetes mellitus. In other preferred embodiments, the postprandial hyperglycemia is a consequence of Type 1 diabetes mellitus or impaired glucose tolerance.

Also featured in the present invention is a pharmaceutical composition. The composition contains a modified exendin or exendin agonist and a pharmaceutically acceptable carrier.

The invention also provides a kit. The kit contains a modified exendin or exendin agonist and instructions and/or packaging for use. The kit may also include a document indicating that the kit, its components, or the methods of using them, has received regulatory approval.

The present invention also provides a method of beneficially regulating gastro-intestinal motility in a subject. The method involves administering to the subject a therapeutically effective amount of a modified exendin or exendin agonist of the present invention.

Also featured are methods of treatment for ingestion of a toxin. The methods involve: (a) administering an amount of a modified exendin or exendin agonist of the present invention effective to prevent or reduce the passage of stomach contents to the intestines; and (b) aspirating the contents of the stomach.

The invention also provides methods for reducing the appetite or weight, or lowering plasma lipids, of a subject, as well as methods for treating gestational diabetes. The invention also provides methods for reducing the appetite or weight, or lowering plasma lipids, of a subject, as well as methods for treating gestational diabetes. Additional methods include modulating triglyceride levels, and treating subjects suffering from dyslipidemia, as well as suppressing glucagon levels. These and other methods of the invention involve administering to the subject a therapeutically effective amount of a modified exendin or exendin agonist of the present invention.

Modified exendins and exendin agonists are useful, for example, as inhibitors of gastric emptying for the treatment of, for example, diabetes mellitus, and obesity. Thus, the present invention is also directed to novel methods for reducing gastric motility and slowing gastric emptying. The methods involve the administration of a modified exendin or exendin agonist, for example one or more PEG polymers linked to exendin-3 [SEQ ID NO. 1], exendin-4 [SEQ ID NO. 2], or other compounds which effectively bind to the receptor at which exendins exert their action on gastric motility and gastric emptying. These methods will be useful in the treatment of, for example, post-prandial hyperglycemia, a complication associated with type 1 (insulin dependent) and type 2 (non-insulin dependent) diabetes mellitus, as well as gestational diabetes, dyslipidemia, to modulate triglyceride levels, and to suppress glucagon secretion.

By "exendin agonist" is meant a compound which mimics the effects of exendins, *e.g*., on gastric motility and gastric emptying (namely, a compound which effectively binds to the receptor at which exendins exert their action on gastric motility and gastric emptying, preferably an analog or derivative of an exendin) or a compound, e.g., that mimics the effects of exendin on the reduction of food intake by binding to the receptor or receptors where exendin causes this effect. Preferred exendin agonist compounds include those described in United States Patent Application Serial No. 90/003,869, entitled, "Use of Exendin And Agonists Thereof For The Reduction of Food Intake", filed January 7, 1998, (and the priority applications thereto) which enjoys common ownership with the present application and which is incorporated by this reference into the present application as though fully set forth herein. Effects of exendins or exendin agonists on reducing food intake can be identified, evaluated, or screened for, using the methods described herein, or other methods known in the art for determining exendin effects, e.g., on food intake or appetite.

In another aspect, a therapeutically effective amount of an amylin agonist is also administered to the subject. In a preferred aspect, the amylin agonist is an amylin or an amylin agonist analog such as ^{25,28,29}Pro-human-amylin. The use of amylin agonists to treat post-prandial hyperglycemia, as well as to beneficially regulate gastrointestinal motility, is described in International Application No. PCT/US94/10225, published March 16, 1995 which has been incorporated by reference herein.

In yet another aspect, a therapeutically effective amount of an insulin or insulin analog is also administered, separately or together with a modified exendin or exendin agonist, to the subject.

Preferably, the subject is a vertebrate, more preferably a mammal, and most preferably a human. In preferred aspects, the modified exendin or exendin agonist of the invention is administered parenterally, more preferably by injection. In a most preferred aspect, the injection is a peripheral injection. Preferably, about 1 µg-30 µg to about 5 mg of the modified exendin or exendin agonist of the invention is administered per day. More preferably, about 1-30 µg to about 2mg, or about 1-30 µg to about 1mg of the modified exendin or exendin agonist of the invention is administered per day. Most preferably, about 3 µg to about 500 µg of the modified exendin or exendin agonist of the invention is administered per day.

Preferred exendins or exendin agonists for modification and use include:
exendin-4 (1-30) [SEQ ID NO 4: His Gly Glu Gly Thr Phe Thr Ser Asp Leu Ser Lys Gln Met Glu Glu Glu Ala Val Arg Leu Phe Ile Glu Trp Leu Lys Asn Gly Gly];
exendin-4 (1-30) amide [SEQ ID NO 5: His Gly Glu Gly Thr Phe Thr Ser Asp Leu Ser Lys Gln Met Glu Glu Glu Ala Val Arg Leu Phe Ile Glu Trp Leu Lys Asn Gly Gly-NH₂];
exendin-4 (1-28) amide [SEQ ID NO 6: His Gly Glu Gly Thr Phe Thr Ser Asp Leu Ser Lys Gln Met Glu Glu Glu Ala Val Arg Leu Phe Ile Glu Trp Leu Lys Asn-NH₂];
¹⁴Leu, ²⁵Phe exendin-4 amide [SEQ ID NO 7: His Gly Glu Gly Thr Phe Thr Ser Asp Leu Ser Lys Gln Leu Glu Glu Glu Ala Val Arg Leu Phe Ile Glu Phe Leu Lys Asn Gly Gly Pro Ser Ser Gly Ala Pro Pro Pro Ser-NH₂];
¹⁴Leu, ²⁵Phe exendin-4 (1-28) amide [SEQ ID NO 8: His Gly Glu Gly Thr Phe Thr Ser Asp Leu Ser Lys Gln Leu Glu Glu Glu Ala Val Arg Leu Phe Ile Glu Phe Leu Lys Asn-NH₂]; and
¹⁴Leu, ²²Ala, ²⁵Phe exendin-4 (1-28) amide [SEQ ID NO 9: His Gly Glu Gly Thr Phe Thr Ser Asp Leu Ser Lys Gln Leu Glu Glu Glu Ala Val Arg Leu Ala Ile Glu Phe Leu Lys Asn-NH₂].

In the methods of the present invention, the modified exendins or exendin agonists may be administered separately or together with one or more other compounds and compositions that exhibit a long term or short-term satiety action, including, but not limited to other compounds and compositions that include an amylin agonist, cholecystokinin (CCK), or a leptin (ob protein). Suitable amylin agonists include, for example, [^{25,28,29}Pro-]-human amylin (also known as "pramlintide," and previously referred to as "AC-137") as described in "Amylin Agonist Peptides and Uses Therefor," U.S. Patent No. 5,686,511, issued November 11, 1997, and salmon calcitonin. The CCK used is preferably CCK octopeptide (CCK-8). Leptin is discussed in, for example, Pelleymounter, M.A., et al. Science 269:540-43 (1995); Halaas, J.L., et al. Science 269:543-46 (1995); and Campfield, L.A., et al. Eur. J. Pharmac. 262:133-41 (1994).

The invention also provides compositions and methods for providing therapeutically effective amounts of the modified exendins or exendin agonists of the invention in order to increase urine flow in an individual, decrease the amount of potassium in the urine of an individual, prevent or alleviate a condition or disorder associated with hypervolemia or toxic hypervolemia in an individual, induce rapid diuresis, prepare an individual for a surgical procedure, increase renal plasma flow and glomerular filtration rates, or treat pre-eclampsia or eclampsia of pregnancy.

### Definitions

In accordance with the present invention and as used herein, the following terms are defined to have the following meanings, unless explicitly stated otherwise.

The term "amino acid" refers to natural amino acids, unnatural amino acids, and amino acid analogs, all in their D and L stereoisomers if their structure allow such stereoisomeric forms. Natural amino acids include alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), Lysine (Lys), methionine (Met), phenylalanine (Phe), proline (Pro), serine (Ser), threonine (Thr), typtophan (Trp), tyrosine (Tyr) and valine (Val). Unnatural amino acids include, but are not limited to azetidinecarboxylic acid, 2-aminoadipic acid, 3-aminoadipic acid, beta-alanine, aminopropionic acid, 2-aminobutyric acid, 4-aminobutyric acid, 6-aminocaproic acid, 2-aminoheptanoic acid, 2-aminoisobutyric acid, 3-aminoisbutyric acid, 2-aminopimelic acid, tertiary-butylglycine, 2,4-diaminoisobutyric acid, desmosine, 2,2'-diaminopimelic acid, 2,3-diaminopropionic acid, N-ethylglycine, N-ethylasparagine, homoproline, hydroxylysine, allo-hydroxylysine, 3-hydroxyproline, 4-hydroxyproline, isodesmosine, allo-isoleucine, N-methylalanine, N-methylglycine, N-methylisoleucine, N-methylpentylglycine, N-methylvaline, naphthalanine, norvaline, norleucine, ornithine, pentylglycine, pipecolic acid and thioproline. Amino acid analogs include the natural and unnatural amino acids which are chemically blocked, reversibly or irreversibly, or modified on their N-terminal amino group or their side chain groups, as for example, methionine sulfoxide, methionine sulfone, S-(carboxymethyl)-cysteine, S-(carboxymethyl)-cysteine sulfoxide and S-(carboxymethyl)-cysteine sulfone.

The term "amino acid analog" refers to an amino acid wherein either the C-terminal carboxy group, the N-terminal amino group or side chain functional group has been chemically codified to another functional group. For example, aspartic acid-(beta-methyl ester) is an amino acid analog of aspartic acid; N-ethylglycine is an amino acid analog of glycine; or alanine carboxamide is an amino acid analog of alanine.

The term "amino acid residue" refers to radicals having the structure: (1) -C(O)-R-NH-, wherein R typically is - CH(R')-, wherein R' is an amino acid side chain, typically H or a carbon containing substitutent;
or (2) 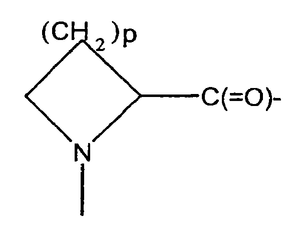 , wherein p is 1, 2, or 3 representing the azetidinecarboxylic acid, proline, or pipecolic acid residues, respectively.

The term "lower" referred to herein in connection with organic radicals such as alkyl groups defines such groups with up to and including about 6, preferably up to and including 4 and advantageously one or two carbon atoms. Such groups may be straight chain or branched chain.

"Pharmaceutically acceptable salt" includes salts of the compounds of the present invention derived from the combination of such compounds and an organic or inorganic acid. In practice the use of the salt form amounts to use of the base form. The compounds of the present invention are useful in both free base and salt form, with both forms being considered as being within the scope of the present invention.

In addition, the following abbreviations stand for the following:
"ACN" or "CH₃CN" refers to acetonitrile.
"Boc", "tBoc" or "Tboc" refers to t-butoxy carbonyl.
"DCC" refers to N,N'-dicyclohexylcarbodiimide.
"Fmoc" refers to fluorenylmethoxycarbonyl.
"HBTU" refers to 2-(1H-benzotriazol-1-yl)-1,1,3,3,-tetramethyluronium hexaflurophosphate.
"HOBt" refers to 1-hydroxybenzotriazole monohydrate.
"homoP" or hPro" refers to homoproline.
"MeAla" or "Nme" refers to N-methylalanine.
"naph" refers to naphthylalanine.
"pG" or pGly" refers to pentylglycine.
"tBuG" refers to tertiary-butylglycine.
"ThioP" or tPro" refers to thioproline.
"3Hyp" refers to 3-hydroxyproline
"4Hyp" refers to 4-hydroxyproline
"NAG" refers to N-alkylglycine
"NAPG" refers to N-alkylpentylglycine
"Norval" refers to norvaline
"Norleu" refers to norleucine

Other features and advantages of the invention will be apparent from the following description of the preferred embodiments thereof, and from the claims.

### BRIEF DESCRIPTION OF THE DRAWINGS

Figure 1 depicts the amino acid sequence for exendin-3 [SEQ. ID. NO. 1] .
Figure 2 depicts the amino acid sequence for exendin-4 [SEQ. ID. NO. 2].

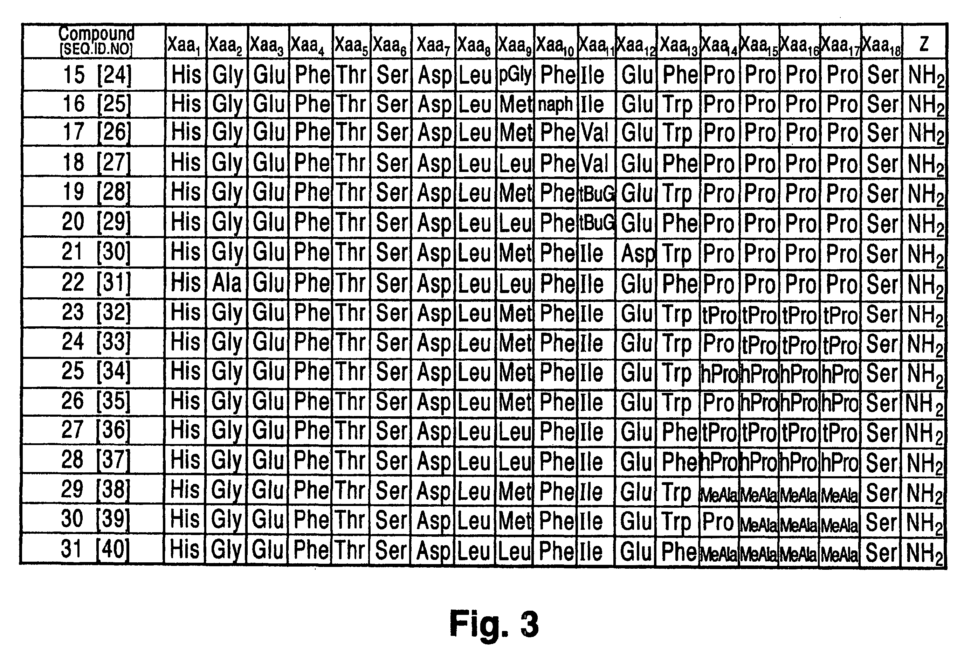

Figure 3 depicts the amino acid sequences for certain exendin agonist compounds useful in the present invention [SEQ. ID. NOS. 10 TO 40].

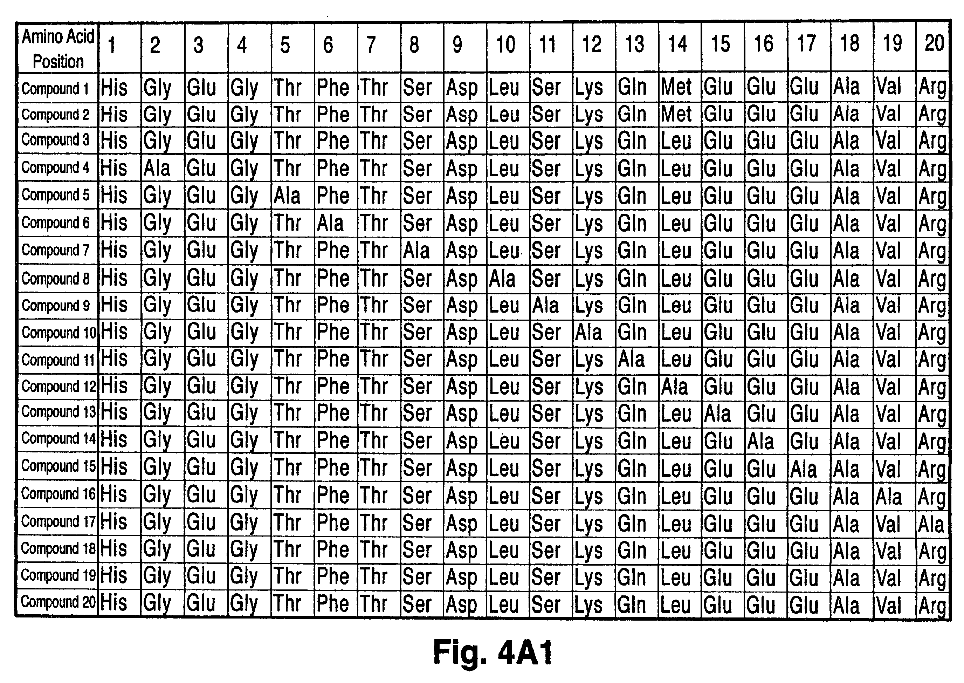

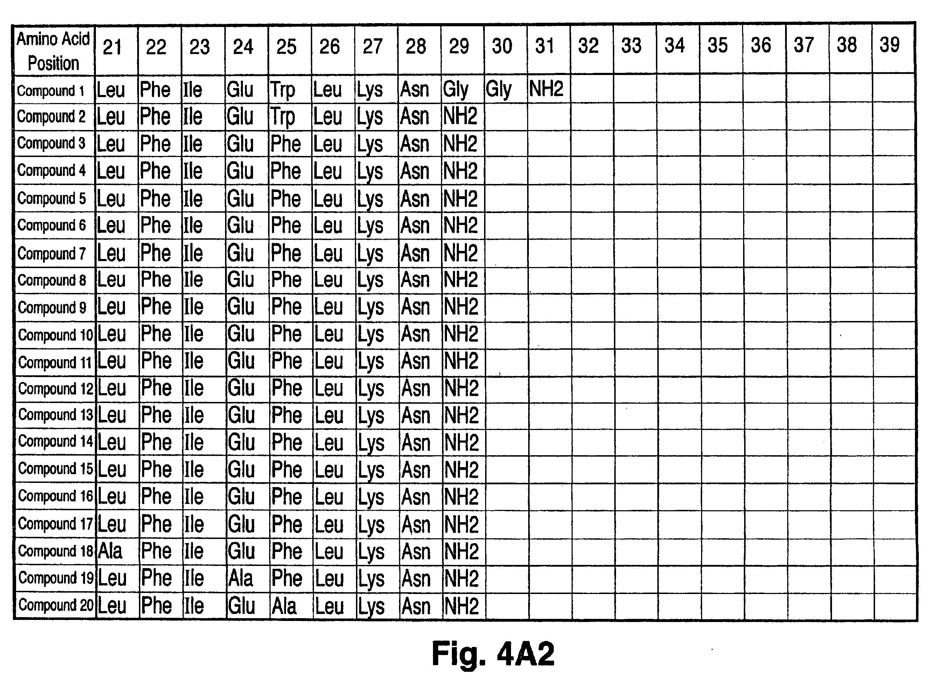

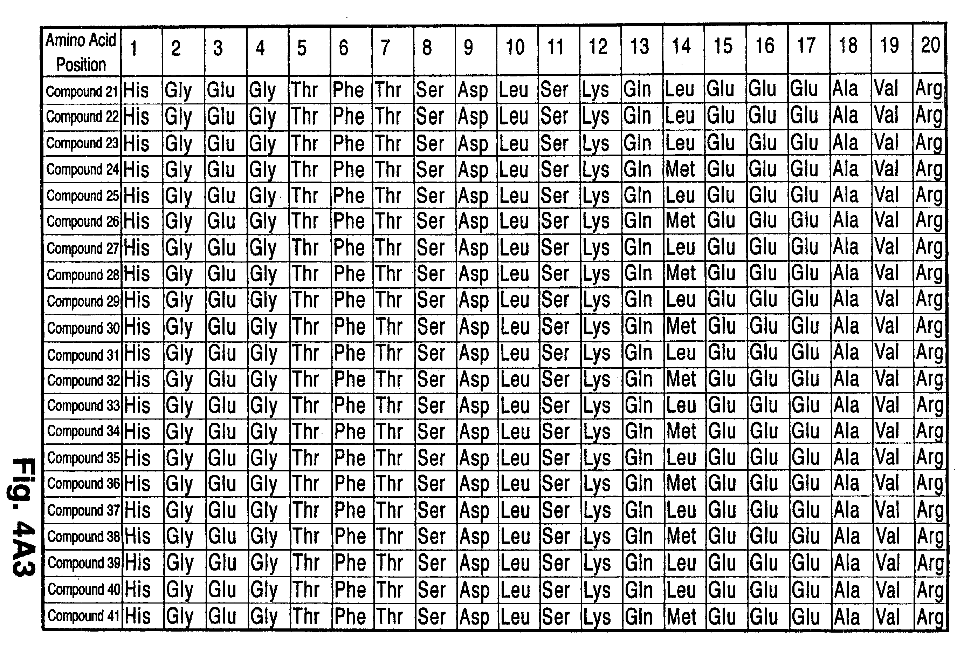

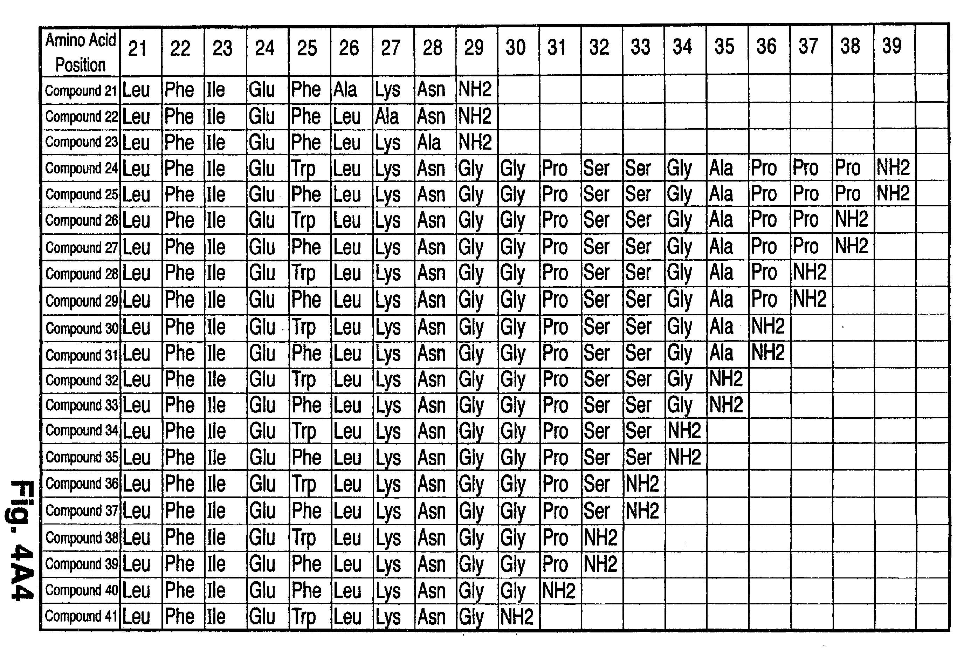

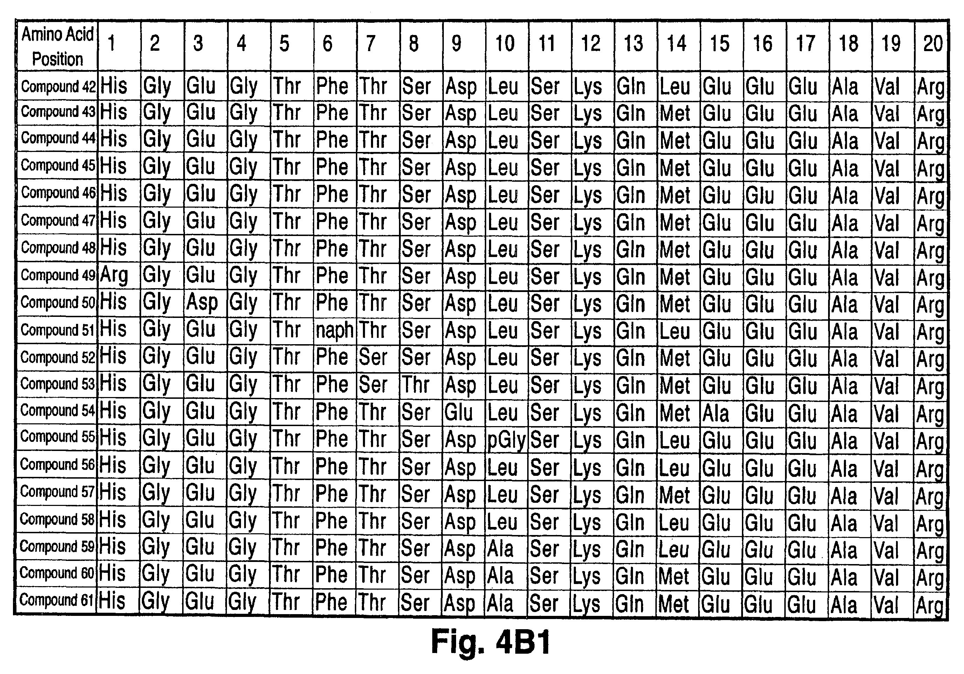

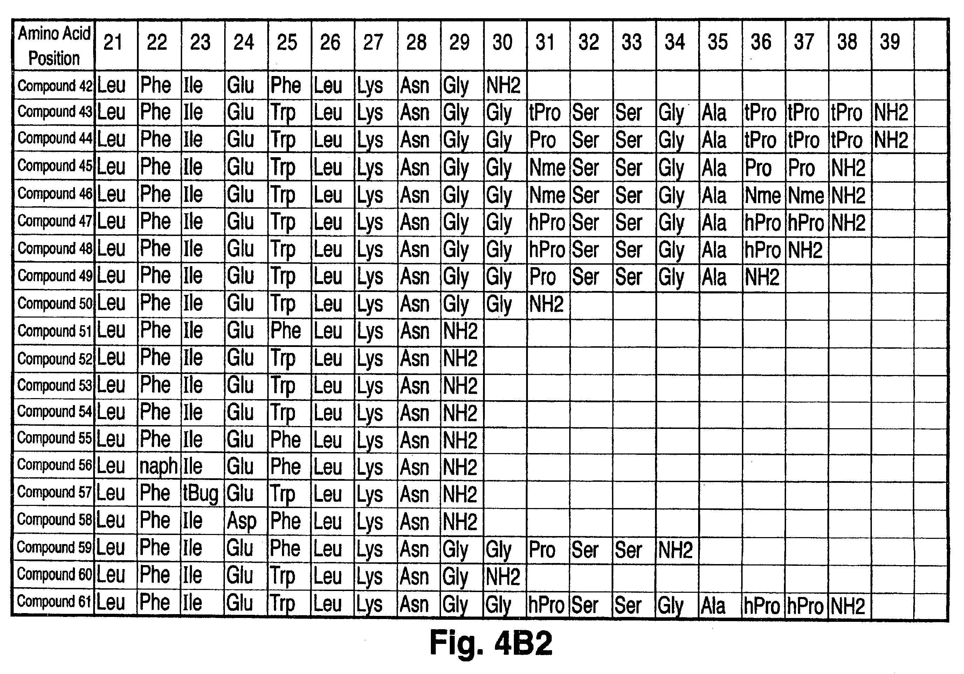

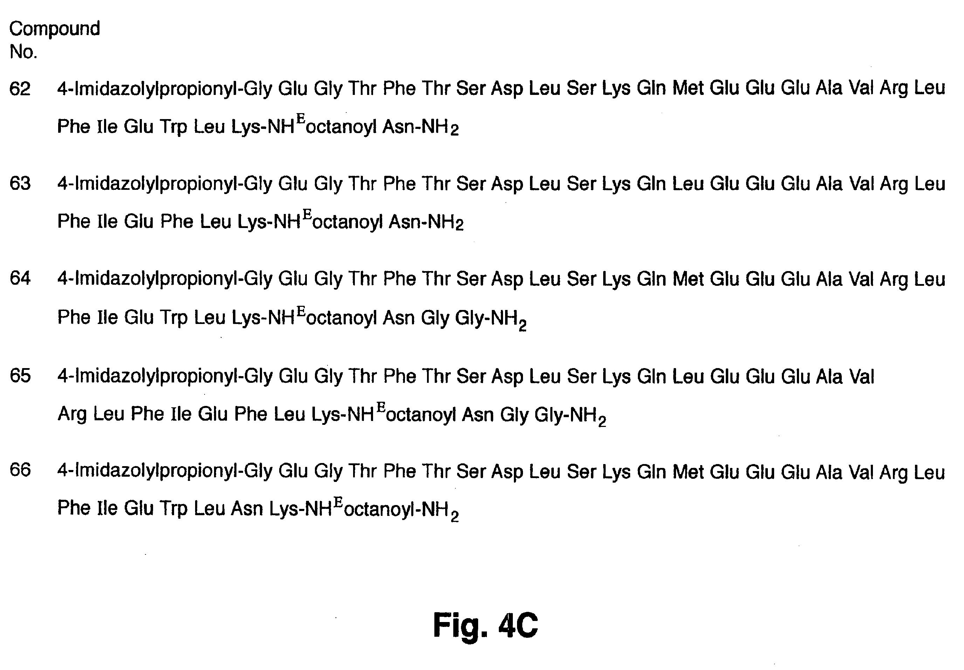

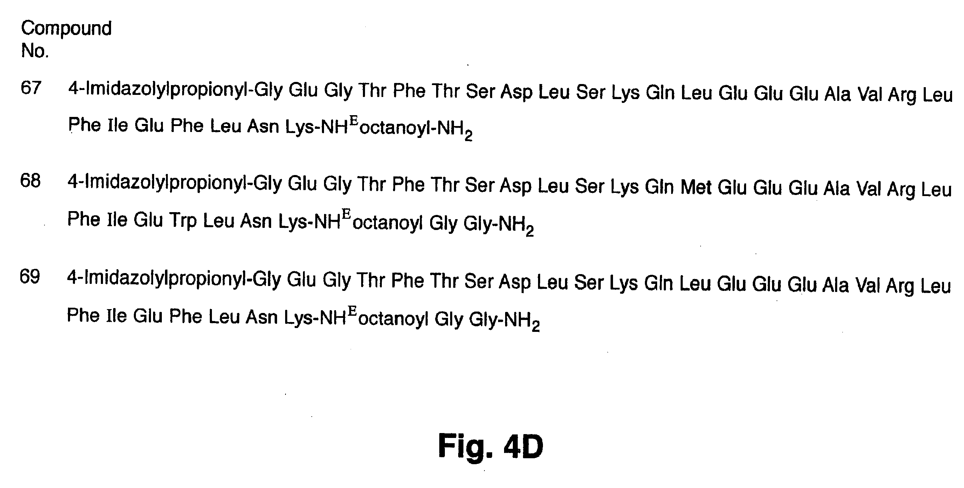

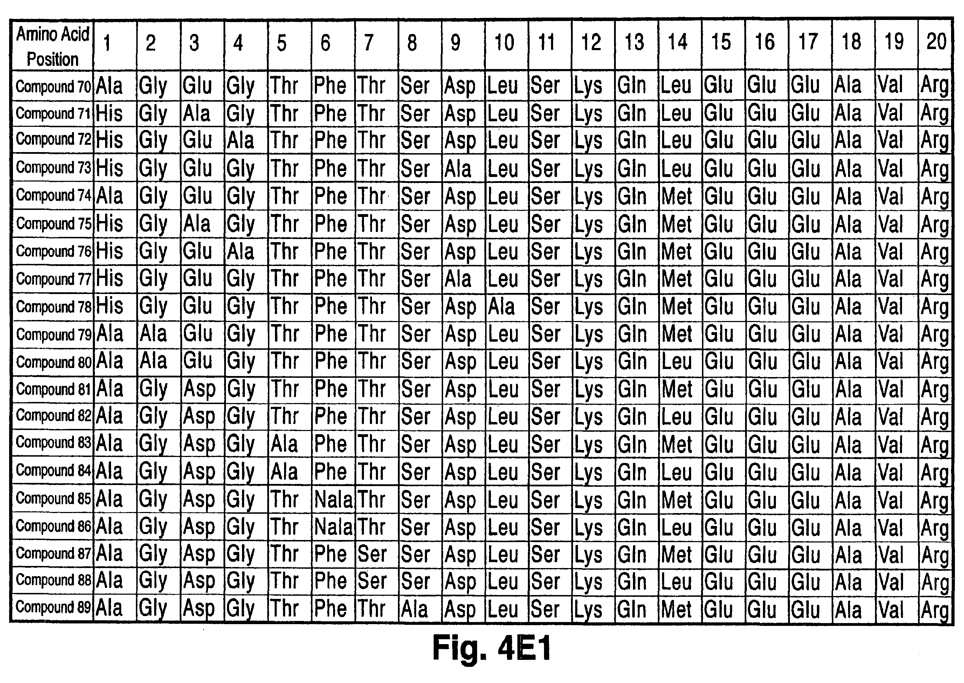

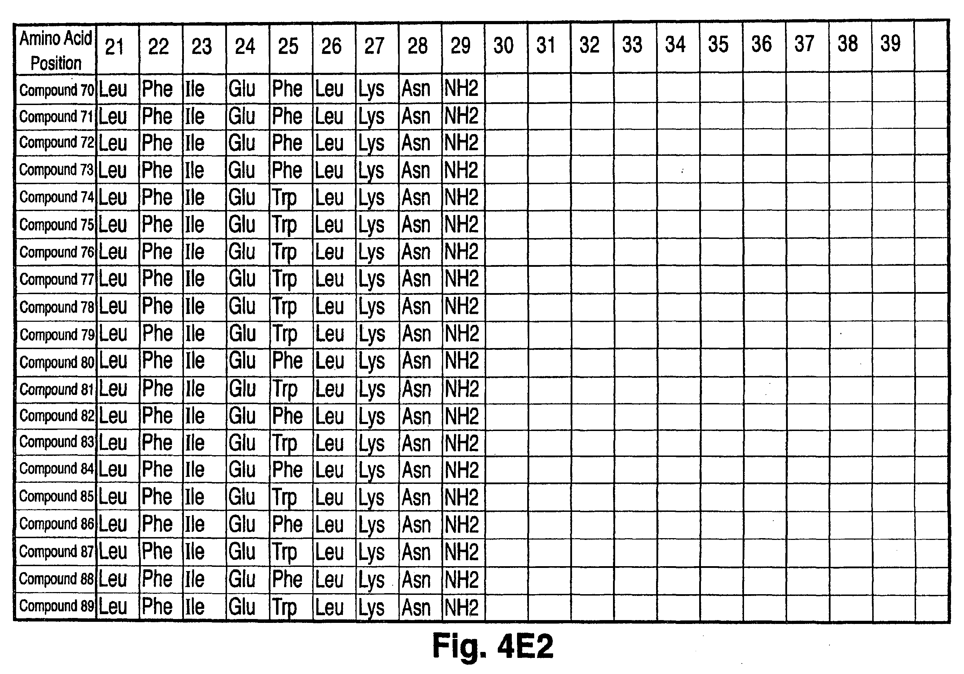

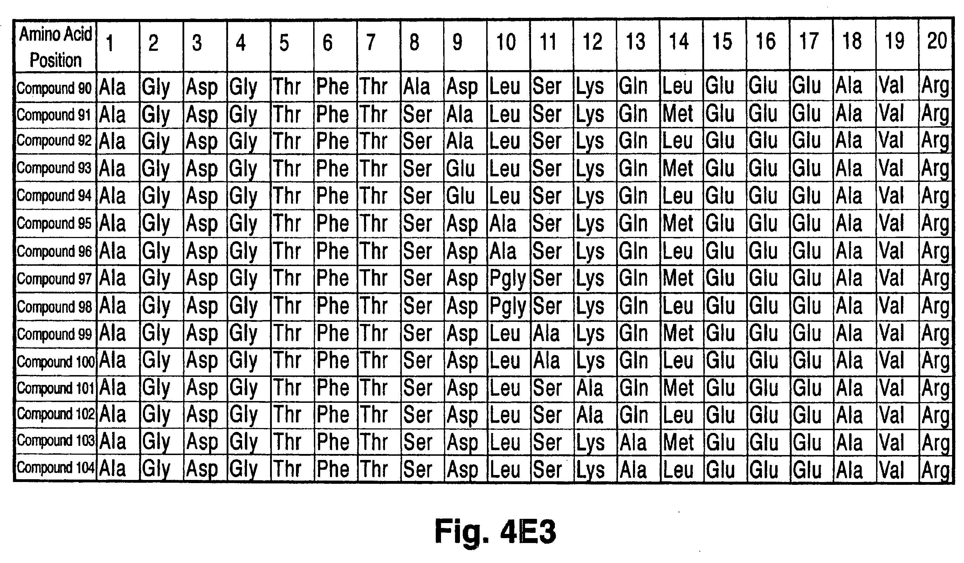

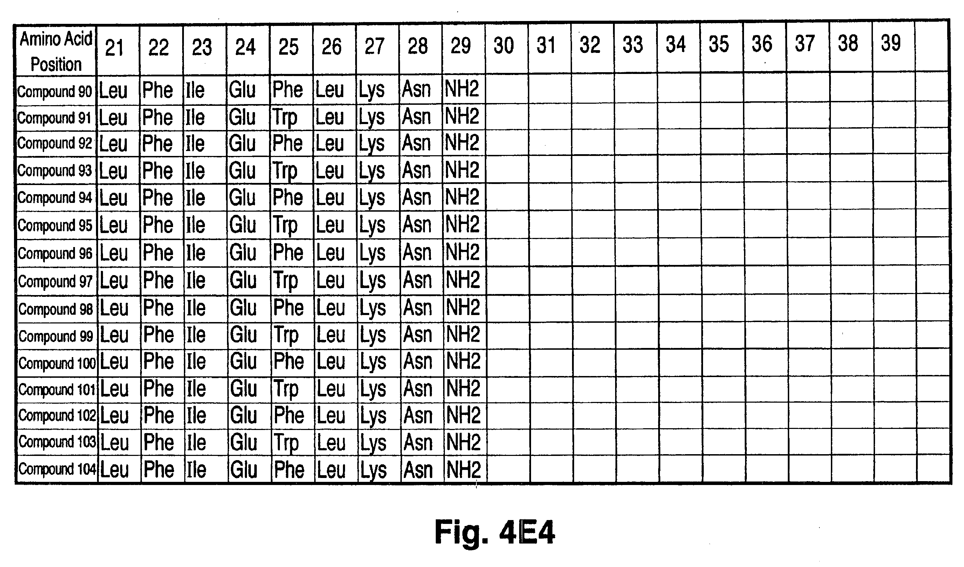

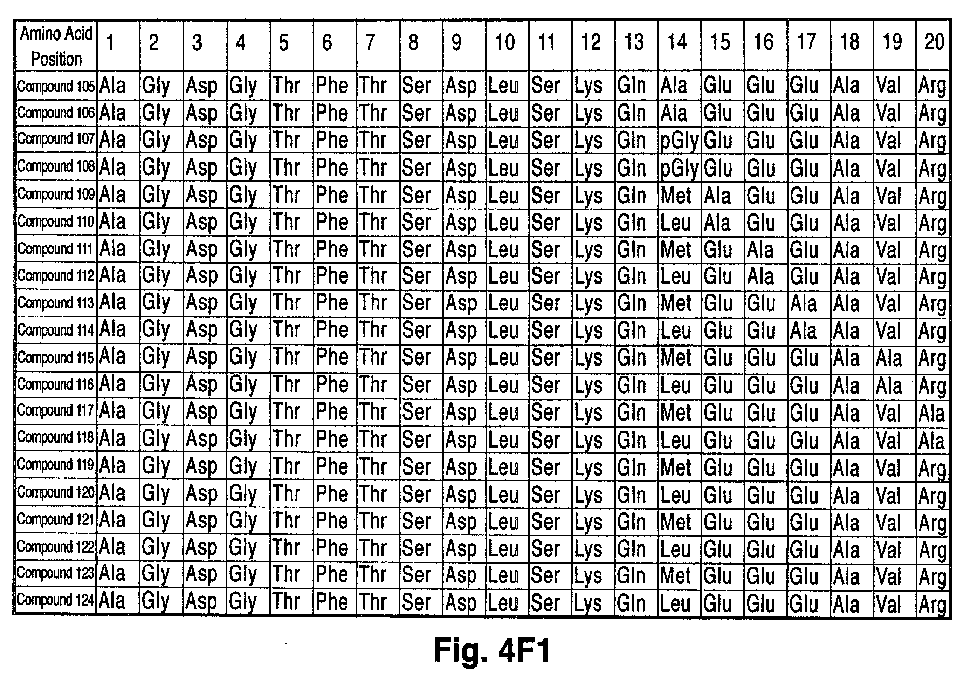

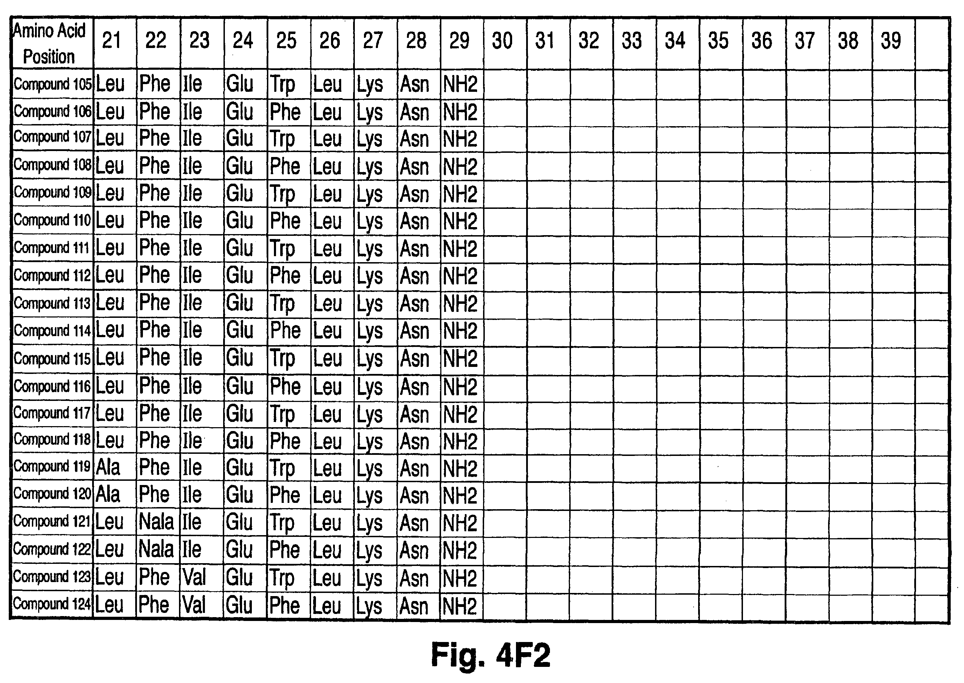

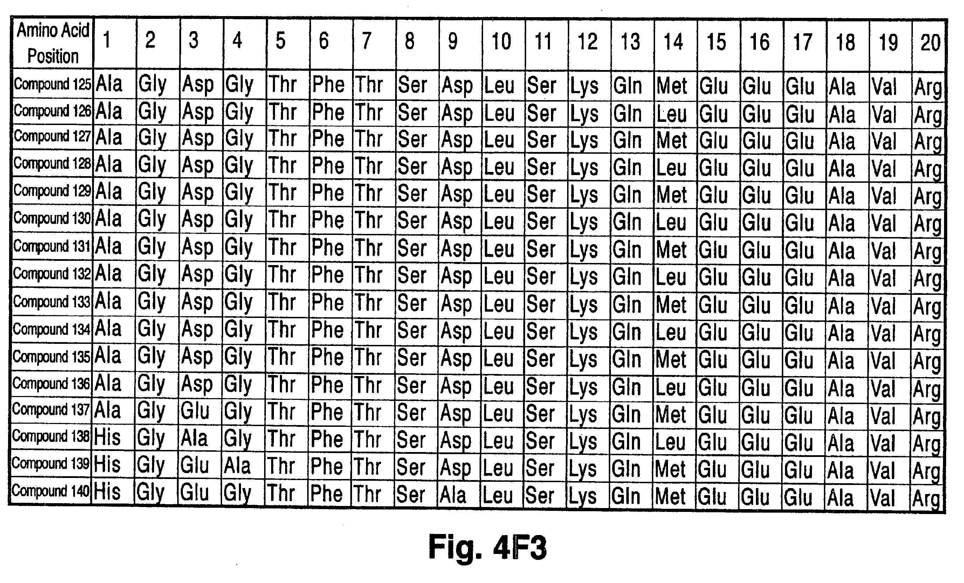

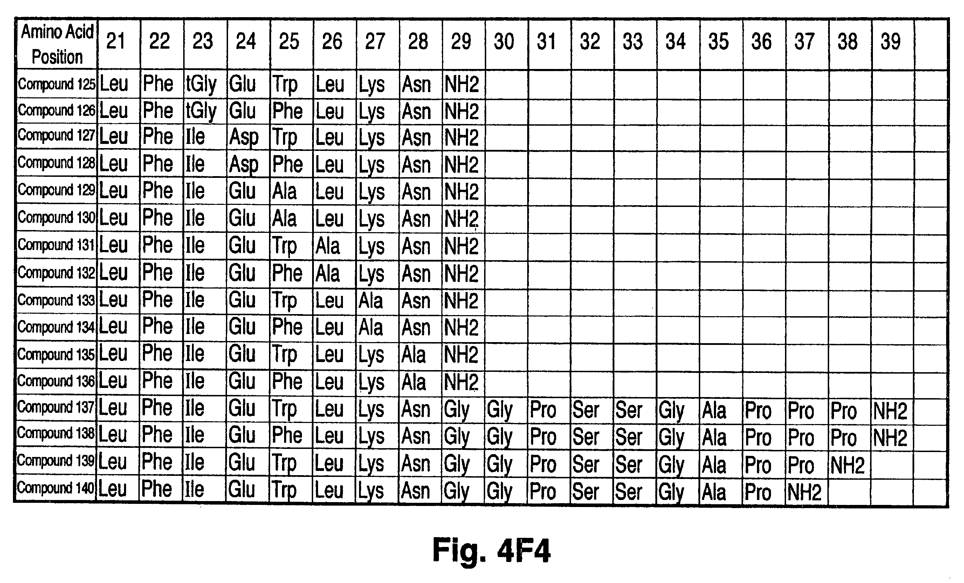

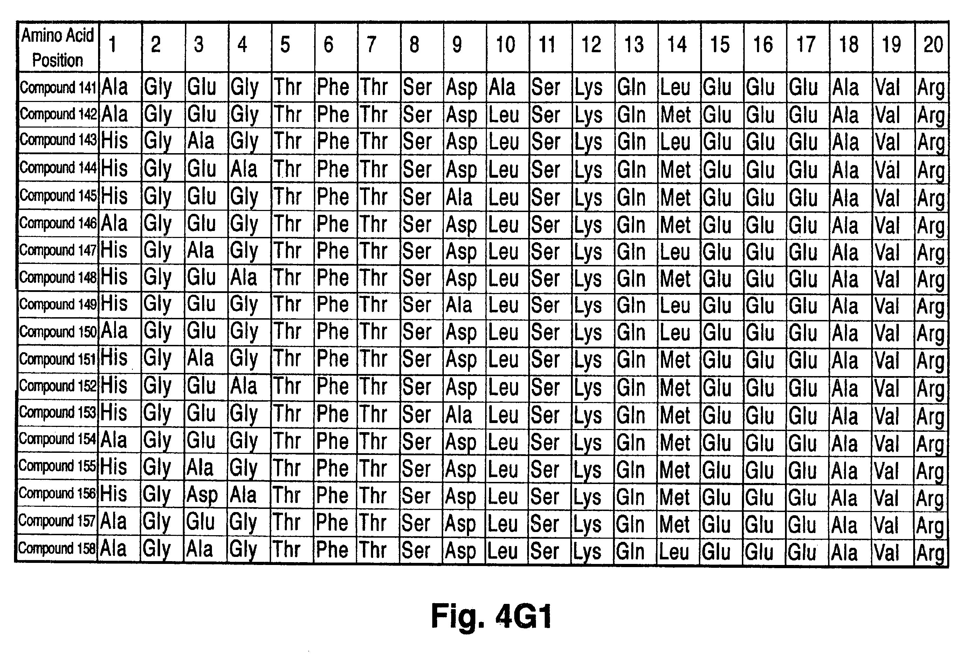

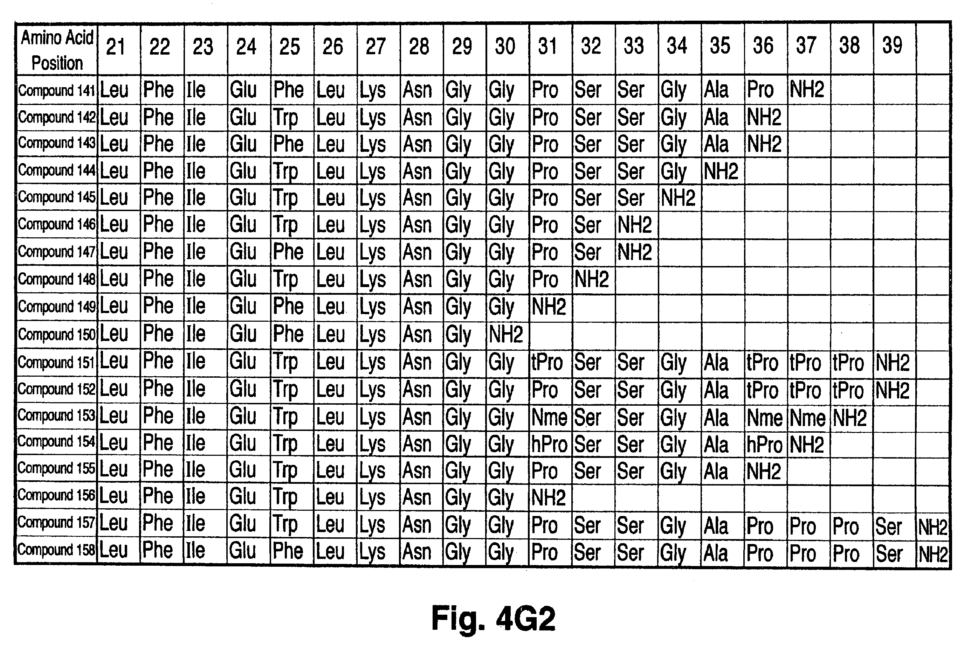

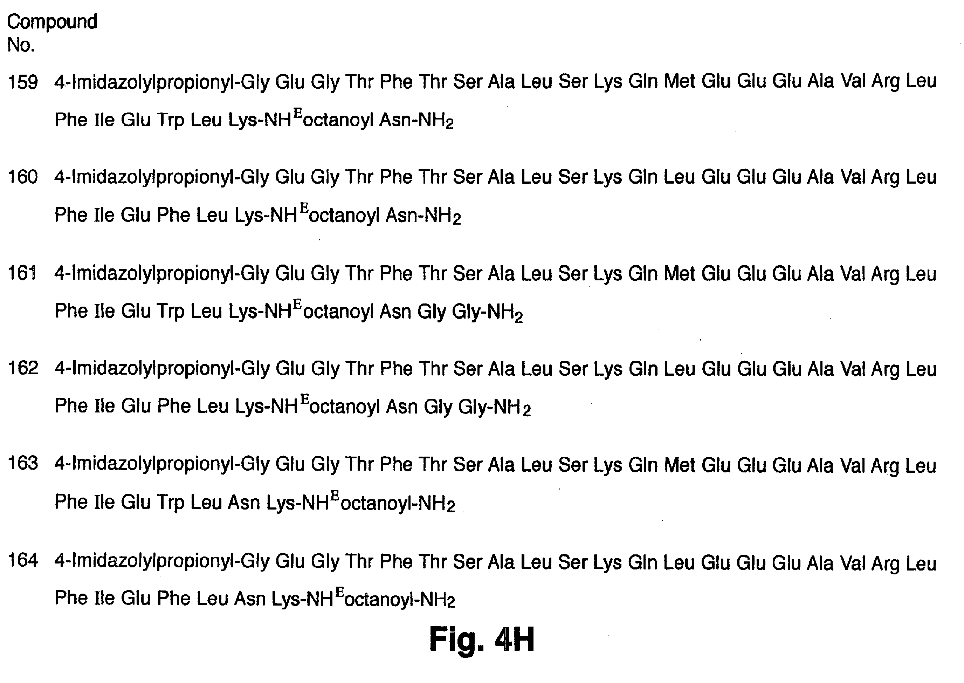

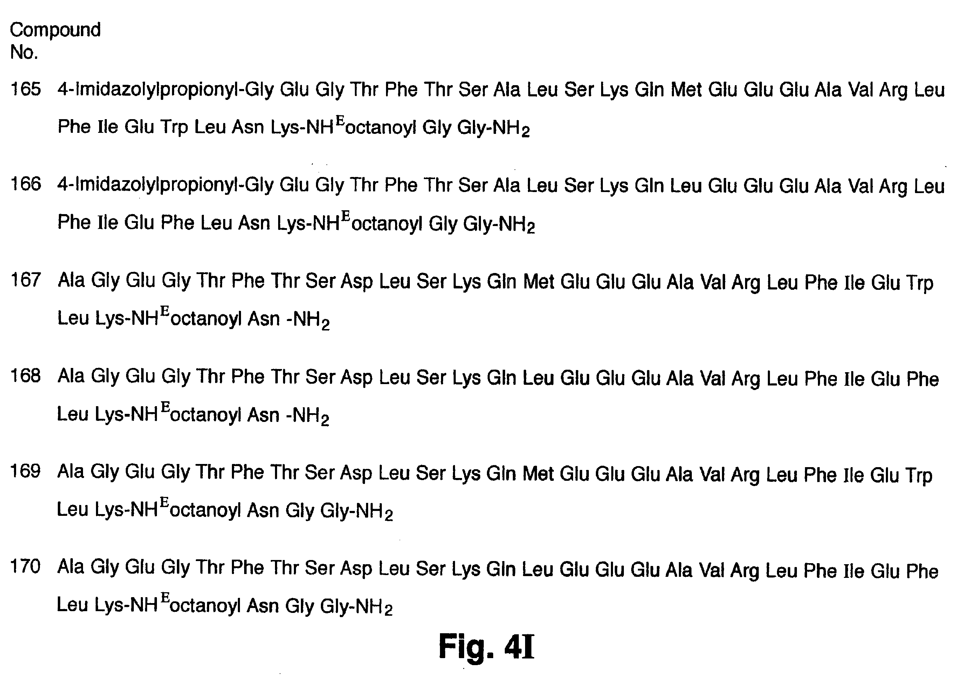

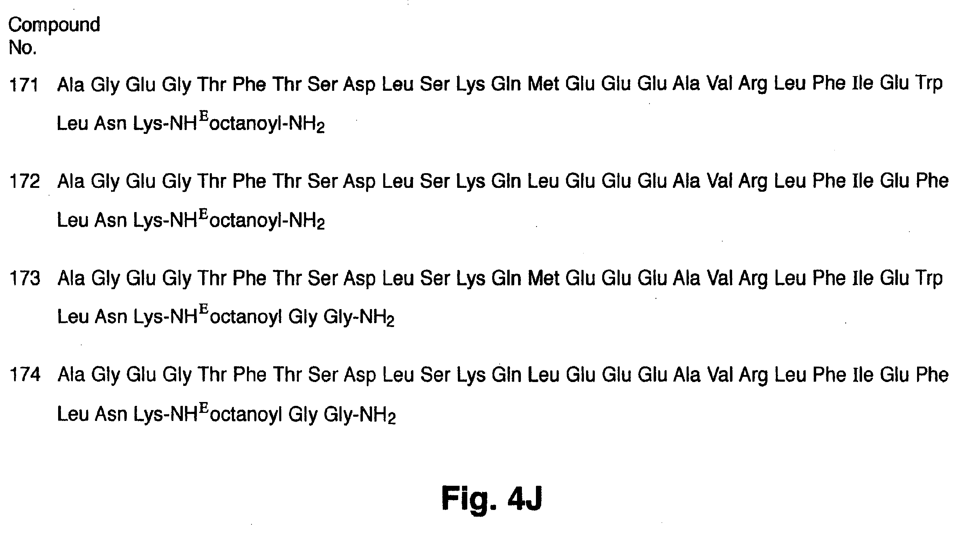

Figure 4 depicts the amino acid sequences for certain compounds of the present invention, Compounds 1-174.
Figure 5 is a graph showing the effect of functional nephrectomy on exendin-4 clearance.
Figure 6 is a graph showing the terminal decay of exendin-4 plasma levels in nephrectomized and sham subjects.

### DETAILED DESCRIPTION OF THE INVENTION

The present invention relates to novel modified exendins and exendin agonists having an exendin or exendin agonist linked to one or more polythylene glycol polymers, and related products and methods that are useful, for example, in the treatment of diabetes, including Type 1, Type 2, and gestational diabetes, in the treatment of disorders which would be benefited by agents which modulate plasma glucose levels or suppress glucagon secretion, and in the treatment of disorders which would be benefited by the administration of agents useful in modulating the rate of gastric emptying or food intake, including obesity, eating disorders, insulin-resistance syndrome, and trigyceride levels, and to treat subjects suffering from dyslipidemia. The methods are also useful for lowering plasma lipid levels, reducing cardiac risk, reducing appetite, and reducing the weight of subjects. Pharmaceutical compositions for use in the methods of the invention are also disclosed.

### Modified Exendins And Exendin Agonists

The modified exendins and exendin agonists of the present invention include one or more PEG polymers linked to an exendin or exendin agonist, such as a naturally occurring exendin, a synthetic exendin or an exendin agonist.

### Exendin-4

Exendin-4 is a naturally occurring peptide isolated from the salivary secretions of the Gila monster. Animal testing of exendin-4 has shown that its ability to lower blood glucose persists for several hours. Exendin-4, a 39-amino acid polypeptide, is synthesized using solid phase synthesis as described herein.

As described herein, the nonclinical pharmacology of exendin-4 has been studied. In the brain, exendin-4 binds principally to the *area postrema* and *nucleus tractus solitarius* region in the hindbrain and to the subfornical organ in the forebrain. Exendin-4 binding has been observed in the rat and mouse brain and kidney. The structures to which exendin-4 binds in the kidney are unknown.

Various experiments have compared the biologic actions of exendin-4 and GLP-1 and demonstrated a more favorable spectrum of properties for exendin-4. A single subcutaneous dose of exendin-4 lowered plasma glucose in db/db (diabetic) and *ob*/*ob* (diabetic obese) mice by up to 40%. In Diabetic Fatty Zucker (ZDF) rats, 5 weeks of treatment with exendin-4 lowered HbA_{1c} (a measure of glycosylated hemoglobin used to evaluate plasma glucose levels) by up to 41%. Insulin sensitivity was also improved by 76% following 5 weeks of treatment in obese ZDF rats. In glucose intolerant primates, dose-dependent decreases in plasma glucose were also observed.

An insulinotropic action of exendin-4 has also been observed in rodents, improving insulin response to glucose by over 100% in non-fasted Harlan Sprague Dawley (HSD) rats, and by up to ∼10-fold in non-fasted *db*/*db* mice. Higher pretreatment plasma glucose concentrations were associated with greater glucose-lowering effects. Thus the observed glucose lowering effect of exendin-4 appears to be glucose-dependent, and minimal if animals are already euglycemic.

Exendin-4 dose dependently slowed gastric emptying in HSD rats and was -90-fold more potent than GLP-1 for this action. Exendin-4 has also been shown to reduce food intake in NIH/Sw (Swiss) mice following peripheral administration, and was at least 1000 times more potent than GLP-1 for this action. Exendin-4 reduced plasma glucagon concentrations by approximately 40% in anesthetized ZDF rats during hyperinsulinemic, hyperglycemic clamp conditions, but did not affect plasma glucagon concentrations during euglycemic conditions in normal rats. Exendin-4 has been shown to dose-dependently reduce body weight in obese ZDF rats, while in lean ZDF rats, the observed decrease in body weight appears to be transient.

Through effects on augmenting and restoring insulin secretion, modified exendins or exendin agonists containing exendin-4, for example, will be useful in people with type 2 diabetes who retain the ability to secrete insulin. Its effects on food intake, gastric emptying, other mechanisms that modulate nutrient absorption, and glucagon secretion also support the utility of such modified exendins and exendin agonists containing exendin-4, for example, in the treatment of, for example, obesity, type 1 diabetes, and people with type 2 diabetes who have reduced insulin secretion.

The toxicology of exendin-4 has been investigated in single-dose studies in mice, rats and monkeys, repeated-dose (up to 28 consecutive daily doses) studies in rats and monkeys and *in vitro* tests for mutagenicity and chromosomal alterations. To date, no deaths have occurred, and there have been no observed treatment-related changes in hematology, clinical chemistry, or gross or microscopic tissue changes. Exendin-4 was demonstrated to be non-mutagenic, and did not cause chromosomal aberrations at the concentrations tested (up to 5000 µg/mL).

In support of the investigation of the nonclinical pharmacokinetics and metabolism of exendin-4, a number of immunoassays have been developed. A radioimmunoassay with limited sensitivity (-100 pM) was used in initial pharmacokinetic studies. A two-site IRMA assay for exendin-4 was subsequently validated with a lower limit of quantitation of 15 pM. The bioavailability of exendin-4, given subcutaneously, was found to be approximately 50-80% using the radioimmunoassay. This was similar to that seen following intraperitoneal administration (48-60%). Peak plasma concentrations (Cₘₐₓ) occurred between 30 and 43 minutes (Tₘₐₓ). Both Cₘₐₓ and AUC values were monotonically related to dose. The apparent terminal half-life for exendin-4 given subcutaneously was approximately 90-110 minutes. This was significantly longer than the 14-41 minutes seen following intravenous dosing. Similar results were obtained using the IRMA assay. Degradation studies with exendin-4 compared to GLP-1 indicate that exendin-4 is relatively resistant to degradation.

### Exendin Agonists

Exendin agonists include exendin peptide analogs in which one or more naturally occurring amino acids are eliminated or replaced with another amino acid(s). Preferred exendin agonists are agonist analogs of exendin-4. Particularly preferred exendin agonists are described in commonly owned PCT Application Serial No. PCT/US98/16387 filed August 6, 1998, entitled "Novel Exendin Agonist Compounds," which claims the benefit of U.S. Patent Application Serial No. 60/055,404, filed August 8, 1997; commonly owned PCT Application Serial No. PCT/US98/24210, filed November 13, 1998, entitled "Novel Exendin Agonist Compounds," which claims the benefit of U.S. Provisional Application No. 60/065,442 filed November 14, 1997; and, commonly owned PCT Application Serial No. PCT/US98/24273, filed November 13, 1998, entitled "Novel Exendin Agonist Compounds," which claims the benefit of U.S. Provisional Application No. 60/066,029 filed November 14, 1997, all of which are incorporated herein by reference in their entirety, including any drawings.

Activity as exendin agonists can be indicated, for example, by activity in the assays described below. Effects of exendins or exendin agonists on gastric motility and gastric emptying can be identified, evaluated, or screened for, using the methods described herein, or other art-known or equivalent methods for determining gastric motility. For example, see U.S. patent application serial no. 60/166,899, entitled, "High Affinity Exendin Receptor," filed November 22, 1999, . Negative receptor assays or screens for exendin agonist compounds or candidate exendin agonist compounds, such as an amylin receptor assay/screen using an amylin receptor preparation as described in U.S. Patent No. 5,264,372, issued November 23, 1993, the contents of which are incorporated herein by reference, one or more calcitonin receptor assays/screens using, for example, T47D and MCF7 breast carcinoma cells, which contain calcium receptors coupled to the stimulation of adenyl cyclase activity, and/or a CGRP receptor assay/screen using, for example, SK-N-MC cells.

One such method for use in identifying or evaluating the ability of a compound to slow gastric motility, involves: (a) bringing together a test sample and a test system, the test sample containing one or more test compounds, the test system containing a system for evaluating gastric motility, the system being characterized in that it exhibits, for example, elevated plasma glucose in response to the introduction to the system of glucose or a meal; and, (b) determining the presence or amount of a rise in plasma glucose in the system. Positive and/or negative controls may be used as well.

Also included within the scope of the present invention are pharmaceutically acceptable salts of the modified compounds of formula (I-VIII) and pharmaceutical compositions including said compounds and salts thereof.

### FORMULA I

Exendin agonist compounds also include those described in U.S. Provisional Application No. 60/065,442, including compounds of the formula (I) [SEQ ID NO. 41]:
Xaa₁ Xaa₂ Xaa₃ Gly Xaa₅ Xaa₆ Xaa₇ Xaa₈ Xaa₉ Xaa₁₀
Xaa₁₁ Xaa₁₂ Xaa₁₃ Xaa₁₄ Xaa₁₅ Xaa₁₆ Xaa₁₇ Ala Xaa₁₉ Xaa₂₀
Xaa₂₁ Xaa₂₂ Xaa₂₃ Xaa₂₄ Xaa₂₅ Xaa₂₆ Xaa₂₇ Xaa₂₈-Z₁; wherein

Xaa₁ is His, Arg or Tyr;
Xaa₂ is Ser, Gly, Ala or Thr;
Xaa₃ is Asp or Glu;
Xaa₅ is Ala or Thr;
Xaa₆ is Ala, Phe, Tyr or naphthylalanine;
Xaa₇ is Thr or Ser;
Xaa₈ is Ala, Ser or Thr;
Xaa₉ is Asp or Glu;
Xaa₁₀ is Ala, Leu, Ile, Val, pentylglycine or Met;
Xaa₁₁ is Ala or Ser;
Xaa₁₂ is Ala or Lys;
Xaa₁₃ is Ala or Gln;
Xaa₁₄ is Ala, Leu, Ile, pentylglycine, Val or Met;
Xaa₁₅ is Ala or Glu;
Xaa₁₆ is Ala or Glu;
Xaa₁₇ is Ala or Glu;
Xaa₁₉ is Ala or Val;
Xaa₂₀ is Ala or Arg;
Xaa₂₁ is Ala or Leu;
Xaa₂₂ is Ala, Phe, Tyr or naphthylalanine;
Xaa₂₃ is Ile, Val, Leu, pentylglycine, tert-butylglycine or Met;
Xaa₂₄ is Ala, Glu or Asp;
Xaa₂₅ is Ala, Trp, Phe, Tyr or naphthylalanine;
Xaa₂₆ is Ala or Leu;
Xaa₂₇ is Ala or Lys;
Xaa₂₈ is Ala or Asn;
Z₁ is-OH,
-NH₂
Gly-Z₂,
Gly Gly-Z₂,
Gly Gly Xaa₃₁-Z₂,
Gly Gly Xaa₃₁ Ser-Z₂,
Gly Gly Xaa₃₁ Ser Ser-Z₂,
Gly Gly Xaa₃₁ Ser Ser Gly-Z₂,
Gly Gly Xaa₃₁ Ser Ser Gly A_{SD-149564.1}Gly Xaa₃₁ Ser Ser Gly Ala Xaa₃₆-Z₂,
Gly Gly Xaa₃₁ Ser Ser Gly Ala Xaa₃₆ Xaa₃₇-Z₂ or
Gly Gly Xaa₃₁ Ser Ser Gly Ala Xaa₃₆ Xaa₃₇ Xaa₃₈-Z₂;
Xaa₃ₗ, Xaa₃₆, Xaa₃₇ and Xaa₃₈ are independently Pro, homoproline, 3Hyp, 4Hyp, thioproline,
N-alkylglycine, N-alkylpentylglycine or
N-alkylalanine; and
Z₂ is -OH or -NH₂;
provided that no more than three of Xaa₃, Xaa₅, Xaa₆, Xaa₈, Xaa₁₀, Xaa₁₁, Xaa₁₂, Xaa₁₃, Xaa₁₉, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ and Xaa₂₈ are Ala. Preferred N-alkyl groups for N-alkylglycine, N-alkylpentylglycine and N-alkylalanine include lower alkyl groups preferably of 1 to about 6 carbon atoms, more preferably of 1 to 4 carbon atoms.

Preferred exendin agonist compounds include those wherein Xaa₁ is His or Tyr. More preferably Xaa₁ is His.

Preferred are those compounds wherein Xaa₂ is Gly.

Preferred are those compounds wherein Xaa₁₄ is Leu, pentylglycine or Met.

Preferred compounds are those wherein Xaa₂₅ is Trp or Phe.

Preferred compounds are those where Xaa₆ is Phe or naphthylalanine; Xaa₂₂ is Phe or naphthylalanine and Xaa₂₃ is Ile or Val.

Preferred are compounds wherein Xaa₃₁, Xaa₃₆, Xaa₃₇ and Xaa₃₈ are independently selected from Pro, homoproline, thioproline and N-alkylalanine.

Preferably Z₁ is -NH₂.

Preferably Z₂ is -NH₂.

According to one aspect, preferred are compounds of formula (I) wherein Xaa₁ is His or Tyr, more preferably His; Xaa₂ is Gly; Xaa₆ is Phe or naphthylalanine; Xaa₁₄ is Leu, pentylglycine or Met; Xaa₂₂ is Phe or naphthylalanine; Xaa₂₃ is Ile or Val; Xaa₃₁, Xaa₃₆, Xaa₃₇ and Xaa₃₈ are independently selected from Pro, homoproline, thioproline or N-alkylalanine. More preferably Z₁ is -NH₂.

According to an especially preferred aspect, especially preferred compounds include those of formula (I) wherein: Xaa₁ is His or Arg; Xaa₂ is Gly or Ala; Xaa₃ is Asp or Glu; Xaa₅ is Ala or Thr; Xaa₆ is Ala, Phe or nephthylalaine; Xaa₇ is Thr or Ser; Xaa₈ is Ala, Ser or Thr; Xaa₉ is Asp or Glu; Xaa₁₀ is Ala, Leu or pentylglycine; Xaa₁₁ is Ala or Ser; Xaa₁₂ is Ala or Lys; Xaa₁₃ is Ala or Gln; Xaa₁₄ is Ala, Leu or pentylglycine; Xaa₁₅ is Ala or Glu; Xaa₁₆ is Ala or Glu; Xaa₁₇ is Ala or Glu; Xaa₁₉ is Ala or Val; Xaa₂₀ is Ala or Arg; Xaa₂₁ is Ala or Leu; Xaa₂₂ is Phe or naphthylalanine; Xaa₂₃ is Ile, Val or tert-butylglycine; Xaa₂₄ is Ala, Glu or Asp; Xaa₂₅ is Ala, Trp or Phe; Xaa₂₆ is Ala or Leu; Xaa₂₇ is Ala or Lys; Xaa₂₈ is Ala or Asn; Z₁ is -OH, -NH₂, Gly-Z₂, Gly Gly-Z₂, Gly Gly Xaa₃₁-Z₂, Gly Gly Xaa₃₁ Ser-Z₂, Gly Gly Xaa₃₁ Ser Ser-Z₂, Gly Gly Xaa₃₁ Ser Ser Gly-Z₂, Gly Gly Xaa₃₁ Ser Ser Gly Ala-Z₂, Gly Gly Xaa₃₁ Ser Ser Gly Ala Xaa₃₆-Z₂, Gly Gly Xaa₃₁ Ser Ser Gly Ala Xaa₃₆ Xaa₃₇-Z₂, Gly Gly Xaa₃₁ Ser Ser Gly Ala Xaa₃₆ Xaa₃₇ Xaa₃₈-Z₂; Xaa₃₁, Xaa₃₆, Xaa₃₇ and Xaa₃₈ being independently Pro homoproline, thioproline or N-methylalanine; and Z₂ being -OH or -NH₂; provided that no more than three of Xaa₃, Xaa₅, Xaa₆, Xaa₈, Xaa₁₀, Xaaₗ₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ and Xaa₂₈ are Ala. Especially preferred compounds include those set forth in PCT application Serial No. PCT/US98/24210, filed November 13, 1998, entitled "Novel Exendin Agonist Compounds" identified therein as compounds 2-23.

According to an especially preferred aspect, provided are compounds where Xaa₁₄ is Leu, Ile, Val or pentylglycine, more preferably Leu or pentylglycine, and Xaa₂₅ is Phe, Tyr or naphthylalanine, more preferably Phe or naphthylalanine. These compounds will be less susceptive to oxidative degration, both *in vitro* and *in vivo,* as well as during synthesis of the compound.

### FORMULA II

Exendin agonist compounds also include those described in U.S. Provisional Application No. 60/066,029, including compounds of the formula (II)[SEQ ID NO. 42]:
Xaa₁ Xaa₂ Xaa₃ Xaa₄ Xaa₅ Xaa₆ Xaa₇ Xaa₈ Xaa₉ Xaa₁₀
Xaa₁₁ Xaa₁₂ Xaa₁₃ Xaa₁₄ Xaa₁₅ Xaa₁₆ Xaa₁₇ Ala Xaa₁₉ Xaa₂ₒ
Xaa₂₁ Xaa₂₂ Xaa₂₃ Xaa₂₄ Xaa₂₅ Xaa₂₆ Xaa₂₇ Xaa₂₈-Z₁; wherein

Xaa₁ is His, Arg, Tyr, Ala, Norval, Val or Norleu;
Xaa₂ is Ser, Gly, Ala or Thr;
Xaa₃ is Ala, Asp or Glu;
Xaa₄ is Ala, Norval, Val, Norleu or Gly;
Xaa₅ is Ala or Thr;
Xaa₆ is Phe, Tyr or naphthylalanine;
Xaa₇ is Thr or Ser;
Xaa₈ is Ala, Ser or Thr;
Xaa₉ is Ala, Norval, Val, Norleu, Asp or Glu;
Xaa₁₀ is Ala, Leu, Ile, Val, pentylglycine or Met;
Xaa₁₁ is Ala or Ser;
Xaa₁₂ is Ala or Lys;
Xaa₁₃ is Ala or Gin;
Xaa₁₄ is Ala, Leu, Ile, pentylglycine, Val or Met;
Xaa₁₅ is Ala or Glu;
Xaa₁₆ is Ala or Glu;
Xaa₁₇ is Ala or Glu;
Xaa₁₉ is Ala or Val;
Xaa₂₀ is Ala or Arg;
Xaa₂₁ is Ala or Leu;
Xaa₂₂ is Phe, Tyr or naphthylalanine;
Xaa₂₃ is Ile, Val, Leu, pentylglycine, tert-butylglycine or Met;
Xaa₂₄ is Ala, Glu or Asp;
Xaa₂₅ is Ala, Trp, Phe, Tyr or naphthylalanine;
Xaa₂₆ is Ala or Leu;
Xaa₂₇ is Ala or Lys;
Xaa₂₈ is Ala or Asn;
   Z₁ is -OH,
      -NH₂,
      Gly-Z₂,
      Gly Gly-Z₂,
      Gly Gly Xaa₃₁-Z₂,
      Gly Gly Xaa₃₁ Ser-Z₂,
      Gly Gly Xaa₃₁ Ser Ser-Z₂,
      Gly Gly Xaa₃₁ Ser Ser Gly-Z₂,
      Gly Gly Xaa₃₁ Ser Ser Gly Ala-2₂,
      Gly Gly Xaa₃₁ Ser Ser Gly Ala Xaa₃₆-Z₂,
      Gly Gly Xaa₃₁ Ser Ser Gly Ala Xaa₃₆ Xaa₃₇-Z₂,
      Gly Gly Xaa₃₁ Ser Ser Gly Ala Xaa₃₆ Xaa₃₇ Xaa₃₈-Z₂ or

      Gly Gly Xaa₃₁ Ser Ser Gly Ala Xaa₃₆ Xaa₃₇ Xaa₃₈ Xaa₃₉-
   Z₂; wherein
   Xaa₃₁, Xaa₃₆, Xaa₃₇ and Xaa₃₈ are independently
   Pro, homoproline, 3Hyp, 4Hyp, thioproline,
   N-alkylglycine, N-alkylpentylglycine or
   N-alkylalanine; and
   Z₂ is -OH or -NH₂;
provided that no more than three of Xaa₃, Xaa₄, Xaa₅, Xaa₆, Xaa₈, Xaa₉, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₉, Xaa₂₅, Xaa₂₆, Xaa₂₇ and Xaa₂₈ are Ala; and provided also that, if Xaa₁ is His, Arg or Tyr, then at least one of Xaa₃, Xaa₄ and Xaa₉ is Ala.

Preferred N-alkyl groups for N-alkylglycine, N-alkylpentylglycine and N-alkylalanine include lower alkyl groups preferably of 1 to about 6 carbon atoms, more preferably of 1 to 4 carbon atoms. Suitable compounds of formula (II) include those described in application Serial No. PCT/US98/24273, filed November 13, 1998, entitled "Novel Exendin Agonist Compounds", identified therein in Examples 1-89 ("Compounds 1-89," respectively), as well as those corresponding compounds identified therein in Examples 104 and 105.

Preferred such exendin agonist compounds include those wherein Xaa₁ is His, Ala or Norval. More preferably Xaa₁ is His or Ala. Most preferably Xaa₁ is His.

Preferred are those compounds of formula (II) wherein Xaa₂ is Gly.

Preferred are those compounds of formula (II) wherein Xaa₃ is Ala.

Preferred are those compounds of formula (II) wherein Xaa₄ is Ala.

Preferred are those compounds of formula (II) wherein Xaa₉ is Ala.

Preferred are those compounds of formula (II) wherein Xaa₁₄ is Leu, pentylglycine or Met.

Preferred compounds of formula (II) are those wherein Xaa₂₅ is Trp or Phe.

Preferred compounds of formula (II) are those where Xaa₆ is Ala, Phe or naphthylalanine; Xaa₂₂ is Phe or naphthylalanine; and Xaa₂₃ is Ile or Val.

Preferred are compounds of formula (II) wherein Xaa₃₁, Xaa₃₆, Xaa₃₇ and Xaa₃₈ are independently selected from Pro, homoproline, thioproline and N-alkylalanine.

Preferably Z₁ is -NH₂.

Preferably Z₂ is -NH₂.

According to one aspect, preferred are compounds of formula (II) wherein Xaa₁ is Ala, His or Tyr, more preferably Ala or His; Xaa₂ is Ala or Gly; Xaa₆ is Phe or naphthylalanine; Xaa₁₄ is Ala, Leu, pentylglycine or Met; Xaa₂₂ is Phe or naphthylalanine; Xaa₂₃ is Ile or Val; Xaa₃₁, Xaa₃₆, Xaa₃₇ and Xaa₃₈ are independently selected from Pro, homoproline, thioproline or N-alkylalanine; and Xaa₃₉ is Ser or Tyr, more preferably Ser. More preferably Z₁ is -NH₂-According to an especially preferred aspect, especially preferred compounds include those of formula (II) wherein: Xaa₁ is His or Ala; Xaa₂ is Gly or Ala; Xaa₃ is Ala, Asp or Glu; Xaa₄ is Ala or Gly; Xaa₅ is Ala or Thr; Xaa₆ is Phe or naphthylalanine; Xaa₇ is Thr or Ser; Xaa₈ is Ala, Ser or Thr; Xaa₉ is Ala, Asp or Glu; Xaa₁₀ is Ala, Leu or pentylglycine; Xaa₁₁ is Ala or Ser; Xaa₁₂ is Ala or Lys; Xaa₁₃ is Ala or Gln; Xaa₁₄ is Ala, Leu, Met or pentylglycine; Xaa₁₅ is Ala or Glu; Xaa₁₆ is Ala or Glu; Xaa₁₇ is Ala or Glu; Xaa₁₉ is Ala or Val; Xaa₂₀ is Ala or Arg; Xaa₂₁ is Ala or Leu; Xaa₂₂ is Phe or naphthylalanine; Xaa₂₃ is Ile, Val or tert-butylglycine; Xaa₂₄ is Ala, Glu or Asp; Xaa₂₅ is Ala, Trp or Phe; Xaa₂₆ is Ala or Leu; Xaa₂₇ is Ala or Lys; Xaa₂₈ is Ala or Asn; Z₁ is -OH, - NH₂, Gly-Z₂, Gly Gly-Z₂, Gly Gly Xaa₃₁-Z₂, Gly Gly Xaa₃₁ Ser-Z₂, Gly Gly Xaa₃₁ Ser Ser-Z₂, Gly Gly Xaa₃₁ Ser Ser Gly-Z₂, Gly Gly Xaa₃₁ Ser Ser Gly Ala-Z₂, Gly Gly Xaa₃₁ Ser Ser Gly Ala Xaa₃₆-Z₂, Gly Gly Xaa₃₁ Ser Ser Gly Ala Xaa₃₆ Xaa₃₇-Z₂, Gly Gly Xaa₃₁ Ser Ser Gly Ala Xaa₃₆ Xaa₃₇ Xaa₃₈-Z₂ or Gly Gly Xaa₃₁ Ser Ser Gly Ala Xaa₃₆ Xaa₃₇ Xaa₃₈ Xaa₃₉-Z₂; Xaa₃₁, Xaa₃₆, Xaa₃₇ and Xaa₃₈ being independently Pro homoproline, thioproline or N-methylalanine; and Z₂ being -OH or -NH₂; provided that no more than three of Xaa₃, Xaa₅, Xaa₆, Xaa₈, Xaa₁₀, Xaa₁₁, Xaa₁₂, Xaa₁₃, Xaa₁₄, Xaa1₅, Xaa₁₆, Xaa₁₇, Xaa₁₉, Xaa₂₀, Xaa₂₁, Xaa₂₄, Xaa₂₅, Xaa₂₆, Xaa₂₇ and Xaa₂₈ are Ala; and provided also that, if Xaa₁ is His, Arg or Tyr, then at least one of Xaa₃, Xaa₄ and Xaa₉ is Ala. Especially preferred compounds of formula (II) include those described in application Serial No. PCT/US98/24273, filed November 13, 1998, entitled "Novel Exendin Agonist Compounds" as having the amino acid sequence of SEQ. ID. NOS. 5-93 therein.

According to an especially preferred aspect, provided are compounds of formula (II) where Xaa₁₄ is Ala, Leu, Ile, Val or pentylglycine, more preferably Leu or pentylglycine, and Xaa₂₅ is Ala, Phe, Tyr or naphthylalanine, more preferably Phe or naphthylalanine. These compounds will be less susceptible to oxidative degration, both *in vitro* and *in vivo*, as well as during synthesis of the compound.

### FORMULA III

Also within the scope of the present invention are narrower genera of compounds having peptides of various lengths, for example genera of compounds which do not include peptides having a length of 28, 29 or 30 amino acid residues, respectively. Additionally, the present invention includes narrower genera of compounds described in PCT application Serial No. PCT/US98/24210, filed November 13, 1998, entitled "Novel Exendin Agonist Compounds" and having particular amino acid sequences, for example, compounds of the formula (III) [SEQ. ID. NO. 43]:
Xaa₁ Xaa₂ Xaa₃ Gly Xaa₅ Xaa₆ Xaa₇ Xaa₈ Xaa₉ Xaa₁₀
Xaa₁₁ Xaa₁₂ Xaa₁₃ Xaa₁₄ Xaa₁₅ Xaa₁₆ Xaa₁₇ Ala Xaa₁₈ Xaa₁₉
Xaa₂₀ Xaa₂₁ Xaa₂₂ Xaa₂₃ Xaa₂₄ Xaa₂₅ Xaa₂₆ Xaa₂₇ Xaa₂₈-Z₁;
wherein
Xaa₁ is His or Arg;
Xaa₂ is Gly or Ala;
Xaa₃ is Asp or Glu;
Xaa₅ is Ala or Thr;
Xaa₆ is Ala, Phe or naphthylalanine;
Xaa₇ is Thr or Ser;
Xaa₈ is Ala, Ser or Thr;
Xaa₉ is Asp or Glu;
Xaa₁₀ is Ala, Leu or pentylglycine;
Xaa₁₁ is Ala or Ser;
Xaa₁₂ is Ala or Lys;
Xaa₁₃ is Ala or Gln;
Xaa₁₄ is Ala, Leu or pentylglycine;
Xaa₁₅ is Ala or Glu;
Xaa₁₆ is Ala or Glu;
Xaa₁₇ is Ala or Glu;
Xaa₁₉ is Ala or Val;
Xaa₂₀ is Ala or Arg;
Xaa₂₁ is Ala or Leu;
Xaa₂₂ is Phe or naphthylalanine;
Xaa₂₃ is Ile, Val or tert-butylglycine;
Xaa₂₄ is Ala, Glu or Asp;
Xaa₂₅ is Ala, Trp, or Phe;
Xaa₂₆ is Ala or Leu;
Xaa₂₇ is Ala or Lys;
Xaa₂₈ is Ala or Asn;
Z₁ is -OH,
   -NH₂,
   Gly-Z₂,
   Gly Gly -Z₂,
   Gly Gly Xaa₃₁-Z₂.
   Gly Gly Xaa₃₁ Ser-Z₂,
   Gly Gly Xaa₃₁ Ser Ser-Z₂,
   Gly Gly Xaa₃₁ Ser Ser Gly-Z₂,
   Gly Gly Xaa₃₁ Ser Ser Gly Ala-Z₂,
   Gly Gly Xaa₃₁ Ser Ser Gly Ala Xaa₃₆-Z₂,
   Gly Gly Xaa₃₁ Ser Ser Gly Ala Xaa₃₆ Xaa₃₇-Z₂ or Gly Gly
   Xaa₃₁ Ser Ser Gly Ala Xaa₃₆ Xaa₃₇ Xaa₃₈-Z₂;
   Xaa₃₁, Xaa₃₆, Xaa₃₇ and Xaa₃₈ are independently selected from the group consisting of Pro,homoproline, thioproline and N-methylylalanine; and
   Z₂ is -OH or -NH₂;
provided that no more than three of Xaa₃, Xaa₅, Xaa₆, Xaa₈, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ and Xaa₂₈ are Ala; and pharmaceutically acceptable salts thereof.

### FORMULA IV

Additionally, the present invention includes narrower genera of peptide compounds described in PCT Application Serial No. PCT/US98/24273, filed November 13, 1998, entitled "Novel Exendin Agonist Compounds" as having particular amino acid sequences, for example, compounds of the formula [IV] [SEQ. ID. NO. 44]:
Xaa₁ Xaa₂ Xaa₃ Xaa₅ Xaa₅ Xaa₆ Xaa₇ Xaa₈ Xaa₉ Xaa₁₀ Xaa₁₁ Xaa₁₂
Xaa₁₃ Xaa₁₄ Xaa₁₅ Xaa₁₆ Xaa₁₇ Ala Xaa₁₈ Xaa₁₉ Xaa₂₀ Xaa₂₁ Xaa₂₂
Xaa₂₃ Xaa₂₄ Xaa₂₅ Xaa₂₆ Xaa₂₇ Xaa₂₈-Z₁; wherein

Xaa₁ is His or Ala;
Xaa₂ is Gly or Ala;
Xaa₃ is Ala, Asp or Glu;
Xaa₄ is Ala or Gly;
Xaa₅ is Ala or Thr;
Xaa₆ is Phe or naphthylalanine;
Xaa₇ is Thr or Ser;
Xaa₈ is Ala, Ser or Thr;
Xaa₉ is Ala, Asp or Glu;
Xaa₁₀ is Ala, Leu or pentylglycine;
Xaa₁₁ is Ala or Ser;
Xaa₁₂ is Ala or Lys;
Xaa₁₃ is Ala or Gln;
Xaa₁₄ is Ala, Leu, Met or pentylglycine;
Xaa₁₅ is Ala or Glu;
Xaa₁₆ is Ala or Glu;
Xaa₁₇ is Ala or Glu;
Xaa₁₉ is Ala or Val;
Xaa₂₀ is Ala or Arg;
Xaa₂₁ is Ala or Leu;
Xaa₂₂ is Phe or naphthylalanine;
Xaa₂₃ is Ile, Val or tert-butylglycine;
Xaa₂₄ is Ala, Glu or Asp;
Xaa₂₅ is Ala, Trp or Phe;
Xaa₂₆ is Ala or Leu;
Xaa₂₇ is Ala or Lys;
Xaa₂₈ is Ala or Asn;
Z₁ is -OH,
   -NH₂,
   Gly-Z₂,
   Gly Gly-Z₂
   Gly Gly Xaa₃₁-Z₂,
   Gly Gly Xaa₃₁ Ser-Z₂,
   Gly Gly Xaa₃₁ Ser Ser-Z₂,
   Gly Gly Xaa₃₁ Ser Ser Gly-Z₂,
   Gly Gly Xaa₃₁ Ser Ser Gly Ala-Z₂,
   Gly Gly Xaa₃₁ Ser Ser Gly Ala Xaa₃₆-Z₂,
   Gly Gly Xaa₃₁ Ser Ser Gly Ala Xaa₃₆ Xaa₃₇-Z₂
   Gly Gly Xaa₃₁ Ser Ser Gly Ala Xaa₃₆ Xaa₃₇ Xaa₃₈-Z₂
   Gly Gly Xaa₃₁ Ser Ser Gly Ala Xaa₃₆ Xaa₃₇ Xaa₃₈
   Ser-Z₂;
   Xaa₃₁, Xaa₃₆, Xaa₃₇ and Xaa₃₈ are independently Pro, homoproline, thioproline, or
   N-methylylalanine; and
   Z₂ is -OH or -NH₂;
provided that no more than three of Xaa₃, Xaa₅, Xaa₆, Xaa₈, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇, and Xaa₂₈ are Ala; and provided that, if Xaa₁ is His, Arg or Tyr, then at least one of Xaa₃, Xaa₄ and Xaa₉ is Ala; and pharmaceutically acceptable salts thereof.

Preferred compounds of formula (IV) include those wherein Xaa₁ is His, Ala, Norval or 4-imidazopropionyl. Preferably, Xaa₁ is His, or 4-imidazopropionyl or Ala, more preferably His or 4-imidazopropionyl.

Preferred compounds of formula (IV) include those wherein Xaa₂ is Gly.

Preferred compounds of formula (IV) include those wherein Xaa₄ is Ala.

Preferred compounds of formula (IV) include those wherein Xaa₉ is Ala.

Preferred compounds of formula (IV) include those wherein Xaa₁₄ is Leu, pentylglycine or Met.

Preferred compounds of formula (IV) include those wherein Xaa₂₅ is Trp or Phe.

Preferred compounds of formula (IV) include those wherein Xaa₆ is Ala, Phe or naphthylalanine; Xaa₂₂ is Phe or naphthylalanine; and Xaa₂₃ is Ile or Val.

Preferred compounds of formula (IV) include those wherein Z₁ is -NH₂.

Preferred compounds of formula (IV) include those wherein Xaa₃₁, Xaa₃₆, Xaa₃₇ and Xaa₃₈ are independently selected from the group consisting of Pro, homoproline, thioproline and N-alkylalanine.

Preferred compounds of formula (IV) include those wherein Xaa₃₉ is Ser or Tyr, preferably Ser.

Preferred compounds of formula (IV) include those wherein Z₂ is -NH₂.

Preferred compounds of formula (IV) include those wherein Z₁ is -NH₂.

Preferred compounds of formula (IV) include those wherein Xaa₂₁ is Lys-NH^{ε}-R where R is Lys, Arg, C₁-C₁₀ straight chain or branched alkanoyl.

Preferred compounds of formula (IV) include those wherein X₁ is Lys Asn, Lys-NH^{ε}-R Asn, or Lys-NH^{ε}-R Ala where R is Lys, Arg, C₁-C₁₀ straight chain or branched alkanoyl. Preferred compounds of formula (IV) include those having an amino acid sequence described in PCT application Serial No. PCT/US98/24273, filed November 13, 1998, entitled "Novel Exendin Agonist Compounds" as being selected from SEQ. ID. NOS. 95-110 therein.

### FORMULA V

Also provided are compounds described in PCT application PCT/US98/24210, filed November 13, 1998, entitled "Novel Exendin Agonist Compounds", including compounds of the formula (V) [SEQ. ID. NO. 45]: 
5 10
Xaa1 Xaa2 Xaa3 Gly Xaa5 Xaa6 Xaa7 Xaa8 Xaa9 Xaa10
Xaa11 Xaa12 Xaa13 Xaa14 Xaa15 Xaa16 Xaa17 Ala Xaa19 Xaa20
Xaa21 Xaa22 Xaa23 Xaa24 Xaa25 Xaa26 X1 -Z1; wherein
Xaa₁ is His, Arg or Tyr or 4-imidazopropionyl;
Xaa₂ is Ser, Gly, Ala or Thr;
Xaa₃ is Asp or Glu;
Xaa₅ is Ala or Thr;
Xaa₆ is Ala, Phe, Tyr or naphthylalanine;
Xaa₇ is Thr or Ser;
Xaa₈ is Ala, Ser or Thr;
Xaa₉ is Asp or Glu;
Xaa₁₀ is Ala, Leu, Ile, Val, pentylglycine or Met;
Xaa₁₁ is Ala or Ser;
Xaa₁₂ is Ala or Lys;
Xaa₁₃ is Ala or Gln;
Xaa₁₄ is Ala, Leu, Ile, pentylglycine, Val or Met;
Xaa₁₅ is Ala or Glu;
Xaa₁₆ is Ala or Glu;
Xaa₁₇ is Ala or Glu;
Xaa₁₉ is Ala or Val;
Xaa₂₀ is Ala or Arg;
Xaa₂₁ is Ala, Leu or Lys-NH^{ε}-R where R is Lys, Arg, C₁-C₁₀ straight chain or branched alkanoyl or cycloalkylalkanoyl;
Xaa₂₂ is Phe, Tyr or naphthylalanine;
Xaa₂₃ is Ile, Val, Leu, pentylglycine, tert-butylglycine or Met;
Xaa₂₄ is Ala, Glu or Asp;
Xaa₂₅ is Ala, Trp, Phe, Tyr or naphthylalanine;
Xaa₂₆ is Ala or Leu;
X₁ is Lys Asn, Asn Lys, Lys-NH^{ε}-R Asn, Asn Lys-NH^{ε}-R, Lys-NH^{ε}-R Ala, Ala Lys-NH^{ε}-R where R is Lys, Arg, C₁-C₁₀ straight chain or branched alkanoyl or cycloalkylalkanoyl
Z₁ is -OH,
   -NH₂,
   Gly-Z₂,
   Gly Gly-Z₂,
   Gly Gly Xaa₃₁-Z₂,
   Gly Gly Xaa₃₁ Ser-Z₂,
   Gly Gly Xaa₃₁ Ser Ser-Z₂,
   Gly Gly Xaa₃₁ Ser Ser Gly-Z₂,
   Gly Gly Xaa₃₁ Ser Ser Gly Ala-Z₂,
   Gly Gly Xaa₃₁ Ser Ser Gly Ala Xaa₃₆-Z₂.
   Gly Gly Xaa₃₁ Ser Ser Gly Ala Xaa₃₆ Xaa₃₇-Z₂ or
   Gly Gly Xaa₃₁ Ser Ser Gly Ala Xaa₃₆ Xaa₃₇ Xaa₃₈-Z₂; wherein
   Xaa₃₁, Xaa₃₆, Xaa₃₇ and Xaa₃₈ are independently selected from the group consisting of Pro, homoproline, 3Hyp, 4Hyp, thioproline,
   N-alkylglycine, N-alkylpentylglycine and
   N-alkylalanine; and
   Z₂ is -OH or -NH₂;
provided that no more than three of Xaa₃, Xaa₅, Xaa₆, Xaa₈, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, and Xaa₂₆ are Ala. Also within the scope of the present invention are pharmaceutically acceptable salts of the compound of formula (V) and
pharmaceutical compositions including said compounds and salts thereof.

Preferred exendin agonist compounds of formula (V) include those wherein Xaa₁ is His, Tyr or 4-imidazopropionyl. More preferably Xaa₁ is His.

Preferred are those compounds of formula (V) wherein Xaa₁ is 4-imidazopropionyl.

Preferred are those compounds of formula (V) wherein Xaa₂ is Gly.

Preferred compounds of formula (V) are those wherein Xaa₁₄ is Leu, pentylglycine or Met.

Preferred compounds of formula (V) are those wherein Xaa₂₅ is Trp or Phe.

According to one aspect, preferred are compounds of formula (V) wherein Xaa₆ is Phe or naphthylalanine; and Xaa₂₂ is Phe or naphthylalanine; and Xaa₂₃ is Ile or Val. More preferably, Z₁ is -NH₂. According to one aspect, especially preferred are such compounds of formula (V) wherein Xaa₃₁, Xaa₃₆, Xaa₃₇ and Xaa₃₈ are independently selected from the group consisting of Pro, homoproline, thioproline and N-alkylalanine. More preferreds, Z₂ is -NH₂.

Preferred compounds of formula (V) include those wherein X₁ is Lys Asn, Lys-NH^{ε}-R Asn, or Lys-NH^{ε}-R Ala where R is Lys, Arg, C₁-C₁₀ straight chain or branched alkanoyl. Preferred compounds of formula (V) include compounds described in PCT application Serial No. PCT/US98/24210, filed November 13, 1998, entitled "Novel Exendin Agonist Compounds" and identified therein as Compound Nos. 62-69.

Preferred such exendin agonist compounds include those wherein Xaa₁ is His, Ala or Norval. More preferably Xaa₁ is His or Ala. Most preferably Xaa₁ is His.

Preferred are those compounds of formula (V) wherein Xaa₂ is Gly.

Preferred are those compounds of formula (V) wherein Xaa₃ is Ala.

Preferred are those compounds of formula (V) wherein Xaa₄ is Ala.

Preferred are those compounds of formula (V) wherein Xaa₉ is Ala.

Preferred are those compounds of formula (V) wherein Xaa₁₄ is Leu, pentylglycine or Met.

Preferred compounds of formula (V) are those wherein Xaa₂₅ is Trp or Phe.

Preferred compounds of formula (V) are those where Xaa₆ is Ala, Phe or naphthylalanine; Xaa₂₂ is Phe or naphthylalanine; and Xaa₂₃ is Ile or Val.

Preferred are compounds of formula (V) wherein Xaa₃₁, Xaa₃₆, Xaa₃₇ and Xaa₃₈ are independently selected from Pro, homoproline, thioproline and N-alkylalanine.

Preferably Z₁ is -NH₂.

Preferably Z₂ is -NH₂.

According to one aspect, preferred are compounds of formula (V) wherein Xaa₁ is Ala, His or Tyr, more preferably Ala or His; Xaa₂ is Ala or Gly; Xaa₆ is Phe or naphthylalanine; Xaa₁₄ is Ala, Leu, pentylglycine or Met; Xaa₂₂ is Phe or naphthylalanine; Xaa₂₃ is Ile or Val; Xaa₃₁, Xaa₃₆, Xaa₃₇ and Xaa₃₈ are independently selected from Pro, homoproline, thioproline or N-alkylalanine; and Xaa₃₉ is Ser or Tyr, more preferably Ser. More preferably Z₁ is -NH₂.

According to an especially preferred aspect, especially preferred compounds include those of formula (V) wherein: Xaa₁ is His or Ala; Xaa₂ is Gly or Ala; Xaa₃ is Ala, Asp or Glu; Xaa₄ is Ala or Gly; Xaa₅ is Ala or Thr; Xaa₆ is Phe or naphthylalanine; Xaa₇ is Thr or Ser; Xaa₈ is Ala, Ser or Thr; Xaa₉ is Ala, Asp or Glu; Xaa₁₀ is Ala, Leu or pentylglycine; Xaa₁₁ is Ala or Ser; Xaa₁₂ is Ala or Lys; Xaa₁₃ is Ala or Gln; Xaa₁₄ is Ala, Leu, Met or pentylglycine; Xaa₁₅ is Ala or Glu; Xaa₁₆ is Ala or Glu; Xaa₁₇ is Ala or Glu; Xaa₁₉ is Ala or Val; Xaa₂₀ is Ala or Arg; Xaa₂₁ is Ala or Leu; Xaa₂₂ is Phe or naphthylalanine; Xaa₂₃ is Ile, Val or tert-butylglycine; Xaa₂₄ is Ala, Glu or Asp; Xaa₂₅ is Ala, Trp or Phe; Xaa₂₆ is Ala or Leu; Xaa₂₇ is Ala or Lys; Xaa₂₈ is Ala or Asn; Z₁ is -OH, - NH₂, Gly-Z₂, Gly Gly-Z₂, Gly Gly Xaa₃₁-Z₂, Gly Gly Xaa₃₁ Ser-Z₂, Gly Gly Xaa₃₁ Ser Ser-Z₂, Gly Gly Xaa₃₁ Ser Ser Gly-Z₂, Gly Gly Xaa₃₁ Ser Ser Gly Ala-Z₂, Gly Gly Xaa₃₁ Ser Ser Gly Ala Xaa₃₆-Z₂, Gly Gly Xaa₃₁ Ser Ser Gly Ala Xaa₃₆ Xaa₃₇-Z₂, Gly Gly Xaa₃₁ Ser Ser Gly Ala Xaa₃₆ Xaa₃₇ Xaa₃₈-Z₂ or Gly Gly Xaa₃₁ Ser Ser Gly Ala Xaa₃₆ Xaa₃₇ Xaa₃₈ Xaa₃₉-Z₂; Xaa₃₁, Xaa₃₆, Xaa₃₇ and Xaa₃₈ being independently Pro homoproline, thioproline or N-methylalanine; and Z₂ being -OH or -NH₂; provided that no more than three of Xaa₃, Xaa₅, Xaa₆, Xaa₈, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, Xaa₂₇ and Xaa₂₈ are Ala; and provided also that, if Xaa₁ is His, Arg or Tyr, then at least one of Xaa₃, Xaa₄ and Xaa₉ is Ala. Especially preferred compounds of formula (V) include those described in PCT application Serial No. PCT/US98/24210, filed November 13, 1998, entitled "Novel Exendin Agonist Compounds" and having the amino acid sequences identified therein as SEQ. ID. NOS. 5-93.

According to an especially preferred aspect, provided are compounds of formula (V) where Xaa₁₄ is Ala, Leu, Ile, Val or pentylglycine, more preferably Leu or pentylglycine, and Xaa₂₅ is Ala, Phe, Tyr or naphthylalanine, more preferably Phe or naphthylalanine. These compounds will be less susceptible to oxidative degration, both *in vitro* and *in vivo,* as well as during synthesis of the compound.

### FORMULA VI

Also provided are peptide compounds described in PCT Application Serial No. PCT/US98/24273, filed November 13, 1998, entitled "Novel Exendin Agonist Compounds", including compounds of the formula (VI) [SEQ. ID. NO. 46]: 
5 10
Xaa1 Xaa2 Xaa3 Xaa4 Xaa5 Xaa6 Xaa7 Xaa8 Xaa9 Xaa10
Xaa11 Xaa12 Xaa13 Xaa14 Xaa15 Xaa16 Xaa17 Ala Xaa19 Xaa20
Xaa21 Xaa22 Xaa23 Xaa24 Xaa25 Xaa26 X1-Z1; wherein
Xaa₁ is His, Arg, Tyr, Ala, Norval, Val, Norleu or 4-imidazopropionyl;
Xaa₂ is Ser, Gly, Ala or Thr;
Xaa₃ is Ala, Asp or Glu;
Xaa₄ is Ala, Norval, Val, Norleu or Gly;
Xaa₅ is Ala or Thr;
Xaa₆ is Phe, Tyr or naphthylalanine;
Xaa₇ is Thr or Ser;
Xaa₈ is Ala, Ser or Thr;
Xaa₉ is Ala, Norval, Val, Norleu, Asp or Glu;
Xaa₁₀ is Ala, Leu, Ile, Val, pentylglycine or Met;
Xaa₁₁ is Ala or Ser;
Xaa₁₂ is Ala or Lys;
Xaa₁₃ is Ala or Gln;
Xaa₁₄ is Ala, Leu, Ile, pentylglycine, Val or Met;
Xaa₁₅ is Ala or Glu;
Xaa₁₆ is Ala or Glu;
Xaa₁₇ is Ala or Glu;
Xaa₁₉ is Ala or Val;
Xaa₂₀ is Ala or Arg;
Xaa₂₁ is Ala, Leu or Lys-NH^{ε}-R where R is Lys, Arg, C¹⁻¹⁰ straight chain or branched alkanoyl or cycloalleyl-alkanoyl;
Xaa₂₂ is Phe, Tyr or naphthylalanine;
Xaa₂₃ is Ile, Val, Leu, pentylglycine, tert-butylglycine or Met;
Xaa₂₄ is Ala, Glu or Asp;
Xaa₂₅ is Ala, Trp, Phe, Tyr or naphthylalanine;
Xaa₂₆ is Ala or Leu;
X₁ is Lys Asn, Asn Lys, Lys-NH^{ε}-R Asn, Asn Lys-NH^{ε}-R, Lys-NH^{ε}-R Ala, Ala Lys-NH^{ε}-R where R is Lys, Arg, C₁-C₁₀ straight chain or branched alkanoyl or cycloalkylalkanoyl
Z₁ is -OH,
   -NH₂,
   Gly-Z₂,
   Gly Gly-Z₂,
   Gly Gly Xaa₃₁-Z₂,
   Gly Gly Xaa₃₁ Ser-Z₂,
   Gly Gly Xaa₃₁ Ser Ser-Z₂,
   Gly Gly Xaa₃₁ Ser Ser Gly-Z₂,
   Gly Gly Xaa₃₁ Ser Ser Gly Ala-Z₂,
   Gly Gly Xaa₃₁ Ser Ser Gly Ala Xaa₃₆-Z₂,
   Gly Gly Xaa₃₁ Ser Ser Gly Ala Xaa₃₆ Xaa₃₇-Z₂,
   Gly Gly Xaa₃₁ Ser Ser Gly Ala Xaa₃₆ Xaa₃₇ Xaa₃₈-Z₂ or
   Gly Gly Xaa₃₁ Ser Ser Gly Ala Xaa₃₆ Xaa₃₇ Xaa₃₈ Xaa₃₉-Z₂;
   wherein
   Xaa₃₁, Xaa₃₆, Xaa₃₇ and Xaa₃₈ are independently selected from the group consisting of Pro, homoproline, 3Hyp, 4Hyp, thioproline,
   N-alkylglycine, N-alkylpentylglycine and
   N-alkylalanine; and
   Z₂ is -OH or -NH₂;
provided that no more than three of Xaa₃, Xaa₄, Xaa₅, Xaa₆, Xaa₈, Xaa₉, Xaa₁₀, Xaa₁₁, Xaa₁₂, Xaa₁₃, Xaa₁₄, Xaa₁₅, Xaa₁₆, Xaa₁₇, Xaa₁₉, Xaa₂₀, Xaa₂₁, Xaa₂₄, Xaa₂₅, Xaa₂₆, are Ala; and provided also that, if Xaa₁ is His, Arg, Tyr, or 4-imidazopropionyl then at least one of Xaa₃, Xaa₄ and Xaa₉ is Ala.

Preferred compounds of formula (VI) include those wherein Xaa₁ is His, Ala, Norval or 4-imidazopropionyl. Preferably, Xaa₁ is His, or 4-imidazopropionyl or Ala, more preferably His or 4-imidazopropionyl.

Preferred compounds of formula (VI) include those wherein Xaa₂ is Gly.

Preferred compounds of formula (VI) include those wherein Xaa₄ is Ala.

Preferred compounds of formula (VI) include those wherein Xaa₉ is Ala.

Preferred compounds of formula (VI) include those wherein Xaa₁₄ is Leu, pentylglycine or Met.

Preferred compounds of formula (VI) include those wherein Xaa₂₅ is Trp or Phe.

Preferred compounds of formula (VI) include those wherein Xaa₆ is Ala, Phe or naphthylalanine; Xaa₂₂ is Phe or naphthylalanine; and Xaa₂₃ is Ile or Val.

Preferred compounds of formula (VI) include those wherein Z₁ is -NH₂-

Preferred compounds of formula (VI) include those wherein Xaa₃₁, Xaa₃₆, Xaa₃₇ and Xaa₃₈ are independently selected from the group consisting of Pro, homoproline, thioproline and N-alkylalanine.

Preferred compounds of formula (VI) include those wherein Xaa₃₉ is Ser or Tyr, preferably Ser.

Preferred compounds of formula (VI) include those wherein Z₂ is -NH₂.

Preferred compounds of formula (VI) include those 42 wherein Z₁ is -NH₂.

Preferred compounds of formula (V-I) include those wherein Xaa₂₁ is Lys-NH^{ε}-R where R is Lys, Arg, C₁-C₁₀ straight chain or branched alkanoyl.

Preferred compounds of formula (VI) include those wherein X₁ is Lys Asn, Lys-NH^{ε}-R Asn, or Lys-NH^{ε}-R Ala where R is Lys, Arg, C₁-C₁₀ straight chain or branched alkanoyl.

Preferred compounds of formula (VI) include those described in PCT Application Serial No. PCT/US98/24273, filed November 13, 1998, entitled "Novel Exendin Agonist Compounds" as having an amino acid sequence selected from those identified therein as SEQ. ID. NOS. 95-110.

### FORMULA VII

Compounds particularly useful according to the present invention are exendin agonist compounds described in U.S. Patent Application Serial No. 09/003,869, filed January 7, 1998, entitled "Use of Exendins And Agonists Thereof For The Reduction of Food Intake", including compounds of the formula (VII) [SEQ. ID. NO. 47]: 
1                           5                          10
Xaa1 Xaa2 Xaa3 Gly Thr Xaa4 Xaa5 Xaa6 Xaa7 Xaa8
                        15                         20
Ser Lys Gln Xaa9 Glu Glu Glu Ala Val Arg Leu
                       25                          30
Xaa10 Xaa11 Xaa12 Xaa13 Leu Lys Asn Gly Gly Xaa14
                  35
Ser Ser Gly Ala Xaa15 Xaa16 Xaa17 Xaa18-Z
 wherein Xaa₁ is His, Arg or Tyr; Xaa₂ is Ser, Gly, Ala or Thr; Xaa₃ is Asp or Glu; Xaa₄ is Phe, Tyr or naphthalanine; Xaa₅ is Thr or Ser; Xaa₆ is Ser or Thr; Xaa₇ is Asp or Glu; Xaa₈ is Leu, Ile, Val, pentylglycine or Met; Xaa₉ is Leu, Ile, pentylglycine, Val or Met; Xaa₁₀ is Phe, Tyr or naphthalanine; Xaa₁₁ is Ile, Val, Leu, pentylglycine, tert-butylglycine or Met; Xaa₁₂ is Glu or Asp; Xaa₁₃ is Trp, Phe, Tyr, or naphthylalanine; Xaa₁₄, Xaa₁₅, Xaa₁₆ and Xaa₁₇ are independently Pro, homoproline, 3Hyp, 4Hyp, thioproline, N-alkylglycine, N-alkylpentylglycine or N-alkylalanine; Xaa₁₈ is Ser, Thr or Tyr; and Z is -OH or -NH₂; with the proviso that the compound does not have the formula of either SEQ. ID. NOS. 1 or 2. Preferred N-alkyl groups for N-alkylglycine, N-alkylpentylglycine and N-alkylalanine include lower alkyl groups preferably of 1 to about 6 carbon atoms, more preferably of 1 to 4 carbon atoms. Suitable compounds include those having amino acid sequences of SEQ. ID. NOS. 10 to 40. Also useful in the present invention are pharmaceutically acceptable salts of the compounds of formula (VII).

Preferred exendin agonist compounds include those wherein Xaa₁ is His or Tyr. More preferably Xaa₁ is His.

Preferred are those compounds wherein Xaa₂ is Gly.

Preferred are those compounds wherein Xaa₉ is Leu, pentylglycine or Met.

Preferred compounds include those wherein Xaa₁₃ is Trp or Phe.

Also preferred are compounds where Xaa₄ is Phe or naphthalanine; Xaa₁₁ is Ile or Val and Xaa₁₄, Xaa₁₅, Xaa₁₆ and Xaa₁₇ are independently selected from Pro, homoproline, thioproline or N-alkylalanine. Preferably N-alkylalanine has a N-alkyl group of 1 to about 6 carbon atoms.

According to an especially preferred aspect, Xaa₁₅, Xaa₁₆ and Xaa₁₇ are the same amino acid reside.

Preferred are compounds wherein Xaa₁₈ is Ser or Tyr, more preferably Ser.

Preferably Z is -NH₂.

According to one aspect, preferred are compounds of formula (VII) wherein Xaa₁ is His or Tyr, more preferably His; Xaa₂ is Gly; Xaa₄ is Phe or naphthalanine; Xaa₉ is Leu, pentylglycine or Met; Xaa₁₀ is Phe or naphthalanine; Xaa₁₁ is Ile or Val; Xaa₁₄, Xaa₁₅, Xaa₁₆ and Xaa₁₇ are independently selected from Pro, homoproline, thioproline or N-alkylalanine; and Xaa₁₈ is Ser or Tyr, more preferably Ser. More preferably Z is -NH₂.

According to an especially preferred aspect, especially preferred compounds include those of formula (VII) wherein: Xaa₁ is His or Arg; Xaa₂ is Gly; Xaa₃ is Asp or Glu; Xaa₄ is Phe or napthylalanine; Xaa₅ is Thr or Ser; Xaa₆ is Ser or Thr; Xaa₇ is Asp or Glu; Xaa₈ is Leu or pentylglycine; Xaa₉ is Leu or pentylglycine; Xaa₁₀ is Phe or naphthylalanine; Xaa₁₁ is Ile, Val or t-butyltylglycine; Xaa₁₂ is Glu or Asp; Xaa₁₃ is Trp or Phe; Xaa₁₄, Xaa₁₅, Xaa₁₆, and Xaa₁₇ are independently Pro, homoproline, thioproline, or N-methylalanine; Xaa₁₈ is Ser or Tyr: and Z is -OH or -NH₂; with the proviso that the compound does not have the formula of either SEQ. ID. NOS. 1 or 2. More preferably Z is -NH₂. Especially preferred compounds include those having the amino acid sequence of SEQ. ID. NOS. 10, 11, 22, 23, 24, 27, 29, 36, 37 and 40.

According to an especially preferred aspect, provided are compounds where Xaa₉ is Leu, Ile, Val or pentylglycine, more preferably Leu or pentylglycine, and Xaa₁₃ is Phe, Tyr or naphthylalanine, more preferably Phe or naphthylalanine. These compounds are believed to exhibit advantageous duration of action and to be less subject to oxidative degration, both *in vitro* and *in vivo,* as well as during synthesis of the compound.

### FORMULA VIII

Also provided are compounds described in PCT Application Serial No. PCT/US98/16387, filed August 6, 1998, entitled "Novel Exendin Agonist Compounds", including compounds of the formula (VIII) [SEQ. ID. NO. 48]: 
1                    5                        10
Xaa1 Xaa2 Xaa3 Gly Thr Xaa4 Xaa5 Xaa6 Xaa7 Xaa8
                    15                       20
Ser Lys Gln Xaa9 Glu Glu Glu Ala Val Arg Leu
 
25                        30
Xaa10 Xaa11 Xaa12 Xaa13 Leu X1 Gly Gly Xaa14
            35
Ser Ser Gly Ala Xaa15 Xaa16 Xaa17 Xaa18-Z
 wherein Xaa₁ is His, Arg, Tyr or 4-imidazopropionyl; Xaa₂ is Ser, Gly, Ala or Thr; Xaa₃ is Asp or Glu; Xaa₄ is Phe, Tyr or naphthylalanine; Xaa₅ is Thr or Ser; Xaa₆ is Ser or Thr; Xaa₇ is Asp or Glu; Xaa₈ is Leu, Ile, Val, pentylglycine or Met; Xaa₉ is Leu, Ile, pentylglycine, Val or Met; Xaa₁₀ is Phe, Tyr or naphthylalanine; Xaa₁₁ is Ile, Val, Leu, pentylglycine, tert-butylglycine or Met; Xaa₁₂ is Glu or Asp; Xaa₁₃ is Trp, Phe, Tyr, or naphthylalanine; X₁ is Lys Asn, Asn Lys, Lys-NH^{ε}-R Asn, Asn Lys-NH^{ε}-R where R is Lys, Arg, C₁-C₁₀ straight chain or branched alkanoyl or cycloalkylalkanoyl; Xaa₁₄, Xaa₁₅, Xaa₁₆ and Xaa₁₇ are independently Pro, homoproline, 3Hyp, 4Hyp, thioproline, N-alkylglycine, N-alkylpentylglycine or N-alkylalanine; Xaa₁₈ is Ser, Thr or Tyr; and Z is -OH or -NH₂; with the proviso that the compound does not have the formula of either SEQ. ID. NOS. 1 or 2. Suitable compounds of formula (VIII) include compounds described in PCT Application Serial No. PCT/US98/16387, filed August 6, 1998, entitled "Novel Exendin Agonist Compounds" having the amino acid sequences of SEQ. ID. NOS. 37-40 therein.

Preferred exendin agonist compounds of formula (VIII) include those wherein Xaa₁ is His, Tyr or 4-imidazopropionyl. More preferably, Xaa₁ is His or 4-imidazopropionyl.

Preferred are those compounds of formula (VIII) wherein Xaa₂ is Gly.

Preferred are those compounds of formula (VIII) wherein Xaa₉ is Leu, pentylglycine or Met.

Preferred are those compounds of formula (VIII) wherein Xaa₁₃ is Trp or Phe.

Preferred are those compounds of formula (VIII) wherein X₁ is Lys Asn, or Lys-NH^{ε}-R Asn, where R is Lys, Arg, C₁-C₁₀ straight chain or branched alkanoyl.

Also preferred are compounds of formula (VIII) wherein Xaa₄ is Phe or naphthylalanine; Xaa₁₀ is Phe or naphthylalanine; Xaa₁₁ is Ile or Val and Xaa₁₄, Xaa₁₅, Xaa₁₆ and Xaa₁₇ are independently selected from Pro, homoproline, thioproline or N-alkylalanine. According to an especially preferred aspect, Xaa₁₈ is Ser or Tyr. Preferred are those such compounds wherein Xaa₁₈ is Ser. Preferably, Z is -NH₂-

According to one preferred aspect, preferred are compounds of formula (VIII) wherein Xaa₄ is Phe or naphthylalanine; Xaa₁₀ is Phe or naphthylalanine; Xaa₁₁ is Ile or Val, X₁ is Lys Asn, or Lys-NH^{ε}-R Asn, where R is Lys, Arg, C₁-C₁₀ straight chain or branched alkanoyl and Xaa₁₄, Xaa₁₅, Xaa₁₆ and Xaa₁₇ are independently selected from Pro, homoproline, thioproline or N-alkylalanine.

### Preparation of Modified Exendins And Exendin Agonists

The modified exendins and exendin agonists of the present invention may be made by linking one or more polyethylene glycol polymers or other molecular weight increasing agents to an exendin or exendin agonist. The synthesis of exendins and exendin agonists is thus described first, followed by methodology for linking the polyethylene glycol polymer(s) to the exendin or exendin agonist.

### Preparation of Exendins And Exendin Agonists

Exendins and exendin agonist compounds such as exendin analogs and exendin derivatives, described herein may be prepared through peptide purification as described in, for example, Eng, et al., J. Biol. Chem. 265:20259-62, 1990; and Eng, et al., J. Biol. Chem. 267:7402-05, 1992, hereby incorporated by reference herein. Alternatively, exendins and exendin agonist peptides may be prepared by methods known to those skilled in the art, for example, as described in Raufman, et al. (J. Biol. Chem. 267:21432-37, 1992), hereby incorporated by reference herein, using standard solid-phase peptide synthesis techniques and preferably an automated or semiautomated peptide synthesizer. The compounds that constitute active ingredients of the formulations and dosages of the present invention may be prepared using standard solid-phase peptide synthesis techniques and preferably an automated or semiautomated peptide synthesizer. Typically, using such techniques, an α-N-carbamoyl protected amino acid and an amino acid attached to the growing peptide chain on a resin are coupled at room temperature in an inert solvent such as dimethylformamide, N-methylpyrrolidinone or methylene chloride in the presence of coupling agents such as dicyclohexylcarbodiimide and 1-hydroxybenzotriazole in the presence of a base such as diisopropylethylamine. The α-N-carbamoyl protecting group is removed from the resulting peptide-resin using a reagent such as trifluoroacetic acid or piperidine, and the coupling reaction repeated with the next desired N-protected amino acid to be added to the peptide chain. Suitable N-protecting groups are well known in the art, with t-butyloxycarbonyl (tBoc) and fluorenylmethoxycarbonyl (Fmoc) being preferred herein.

The solvents, amino acid derivatives and 4-methylbenzhydryl-amine resin used in the peptide synthesizer may be purchased from Applied Biosystems Inc. (Foster City, CA). The following side chain-protected amino acids may be purchased from Applied Biosystems, Inc.: BSD-112344.1-Arg(Pmc), Boc-Thr(Bzl), Fmoc-Thr(t-Bu), Boc-Ser(Bzl), Fmoc-Ser(t-Bu), Boc-Tyr(BrZ), Fmoc-Tyr(t-Bu), Boc-Lys(C1-Z), Fmoc-Lys(Boc), Boc-Glu(Bzl), Fmoc-Glu(t-Bu), Fmoc-His(Trt), Fmoc-Asn(Trt), and Fmoc-Gln(Trt). Boc-His(BOM) may be purchased from Applied Biosystems, Inc. or Bachem Inc. (Torrance, CA). Anisole, dimethylsulfide, phenol, ethanedithiol, and thioanisole may be obtained from Aldrich Chemical Company (Milwaukee, WI). Air Products and Chemicals (Allentown, PA) supplies HF. Ethyl ether, acetic acid and methanol may be purchased from Fisher Scientific (Pittsburgh, PA).

Solid phase peptide synthesis may be carried out with an automatic peptide synthesizer (Model 430A, Applied Biosystems Inc., Foster City, CA) using the NMP/HOBt (Option 1) system and tBoc or Fmoc chemistry (see, Applied Biosystems User's Manual for the ABI 430A Peptide Synthesizer, Version 1.3B July 1, 1988, section 6, pp. 49-70, Applied Biosystems, Inc., Foster City, CA) with capping. Boc-peptide-resins may be cleaved with HF (-50°C to 0°C, 1 hour). The peptide may be extracted from the resin with alternating water and acetic acid, and the filtrates lyophilized. The Fmoc-peptide resins may be cleaved according to standard methods (Introduction to Cleavage Techniques, Applied Biosystems, Inc., 1990, pp. 6-12). Peptides may also be assembled using an Advanced Chem Tech Synthesizer (Model MPS 350, Louisville, Kentucky).

Peptides may be purified by RP-HPLC (preparative and analytical) using a Waters Delta Prep 3000 system. A C4, C8 or C18 preparative column (10 µ, 2.2 x 25 cm; Vydac, Hesperia, CA) may be used to isolate peptides, and purity may be determined using a C4, C8 or C18 analytical column (5 µ, 0.46 x 25 cm; Vydac). Solvents (A-0.1% TFA/water and B=0.1% TFA/CH₃CN) may be delivered to the analytical column at a flowrate of 1.0 ml/min and to the preparative column at 15 ml/min. Amino acid analyses may be performed on the Waters Pico Tag system and processed using the Maxima program. Peptides may be hydrolyzed by vapor-phase acid hydrolysis (115°C, 20-24 h). Hydrolysates may be derivatized and analyzed by standard methods (Cohen, et al., The Pico Tag Method: A Manual of Advanced Techniques for Amino Acid Analysis, pp. 11-52, Millipore Corporation, Milford, MA (1989)). Fast atom bombardment analysis may be carried out by M-Scan, Incorporated (West Chester, PA). Mass calibration may be performed using cesium iodide or cesium iodide/glycerol. Plasma desorption ionization analysis using time of flight detection may be carried out on an Applied Biosystems Bio-Ion 20 mass spectrometer. Electrospray mass spectroscopy may be carried and on a VG-Trio machine.

Peptide active ingredient compounds useful in the formulations and dosages of the invention may also be prepared using recombinant DNA techniques, using methods now known in the art. See, *e.g*., Sambrook et al., Molecular Cloning: A Laboratory Manual, 2d Ed., Cold Spring Harbor (1989). Alternatively, such compounds may be prepared by homogeneous phase peptide synthesis methods. Non-peptide compounds useful in the present invention may be prepared by art-known methods. For example, phosphate-containing amino acids and peptides containing such amino acids, may be prepared using methods known in the art. See, *e.g*., Bartlett and Landen, Biorg. Chem. 14:356-377 (1986).

### Conjugation of polyethylene glycol polymers (or other molecular weight increasing agents)

There are several strategies for coupling PEG to peptides/proteins. See, Int. J. Hematology 68:1 (1998); Bioconjugate Chem. 6:150 (1995) ; and Crit. Rev. Therap. Drug Carrier Sys. 9:249 (1992) all of which are incorporated herein by reference in their entirety. Those skilled in the art, therefore, will be able to utilize such well known techniques for linking one or more polethylene glycol polymers to the exendins and exendin agonists described herein. Suitable polethylene glycol polymers typically are commercially available or may be made by techniques well known to those skilled in the art. The polyethylene glycol polymers or other molecular weight increasing agents preferably have molecular weights between 500 and 20,000 and may be branched or straight chain polymers.

The attachment of a PEG on an intact peptide or protein can be accomplished by coupling to amino, carboxyl or thiol groups. These groups will typically be the N and C termini and on the side chains of such naturally occurring amino acids as lysine, aspartic acid, glutamic acid and cysteine. Since exendin-4 and other exendins and exendin agonists can be prepared by solid phase peptide chemistry techniques, a variety of moieties containing diamino and dicarboxylic groups with orthogonal protecting groups can be introduced for conjugation to PEG.

The present invention also provides for conjugation of an exendin or exendin agonist to one or more polymers other than polyethylene glycol which can regulate kidney clearance in a manner similar to polyethylene glycol. Examples of such polymers include albumin and gelatin. See, Gombotz and Pettit, Bioconjugate Chem., 6:332-351, 1995, which is incorporated herein by reference in its entirety.

### Utility

The formulations and dosages described herein are useful in view of their pharmacological properties. In particular, the compounds of the invention possess activity as agents to reduce food intake and as agents to regulate gastric motility and to slow gastric emptying, as evidenced by the ability to inhibit gastric emptying levels in mammals. They can be used to treat conditions or diseases which can be alleviated by reducing food intake or regulating gastric motility. The formulations and dosages of the invention are also effective as exendins and exendin agonists, and possess activity as agents to lower blood glucose, and to regulate gastric motility and to slow gastric emptying, as evidenced by the ability to reduce post-prandial glucose levels in mammals. The compounds of the present invention are useful in *in vitro* and *in vivo* scientific methods for investigation of exendins and exendin agonists for example in methods such as those described herein.

The compounds referenced above may form salts with various inorganic and organic acids and bases. Such salts include salts prepared with organic and inorganic acids, for example, HCl, HBr, H₂SO₄, H₃PO₄, trifluoroacetic acid, acetic acid, formic acid, methanesulfonic acid, toluenesulfonic acid, maleic acid, fumaric acid and camphorsulfonic acid. Salts prepared with bases include ammonium salts, alkali metal salts, *e.g*., sodium and potassium salts, and alkali earth salts, *e.g*., calcium and magnesium salts. Acetate, hydrochloride, and trifluoroacetate salts are preferred. The salts may be formed by conventional means, as by reacting the free acid or base forms of the product with one or more equivalents of the appropriate base or acid in a solvent or medium in which the salt is insoluble, or in a solvent such as water which is then removed *in vacuo* or by freeze-drying or by exchanging the ions of an existing salt for another ion on a suitable ion exchange resin.

### Formulation and Administration

Modified exendin and exendin agonist formulations and dosages of the invention are useful in view of their exendin-like effects, and may conveniently be provided in the form of formulations suitable for parenteral (including intravenous, intramuscular and subcutaneous) administration. Also described herein are formulations and dosages useful in alternative delivery routes, including oral, nasal, buccal, sublingual and pulmonary.

The feasibility of alternate routes of delivery for exendin-4 has been explored by measuring exendin-4 in the circulation in conjunction with observation of a biologic response, such as plasma glucose lowering in diabetic animals, after administration. Passage of exendin-4 has been investigated across several surfaces, the respiratory tract (nasal, tracheal and pulmonary routes) and the gut (sublingual, gavage and intraduodenal routes). Biologic effect and appearance of exendin-4 in blood have been observed with each route of administration via the respiratory tract, and with sublingual and gavaged peptide via the gastrointestinal tract. Intra-tracheal administration, nasal administration, administration via the gut, and sublingual administration have all been described.

In some cases, it will be convenient to provide a modified exendin or exendin agonist and another anti-gastric-emptying agent, such as glucagon, an amylin, or an amylin agonist, in a single composition or solution for administration together. In other cases, it may be more advantageous to administer another anti-emptying agent separately from the modified exendin or exendin agonist. In yet other cases, it may be beneficial to provide a modified exendin or exendin agonist either co-formulated or separately with other glucose lowering agents such as insulin. A suitable administration format may best be determined by a medical practitioner for each patient individually. Suitable pharmaceutically acceptable carriers and their formulation are described in standard formulation treatises, *e.g*., Remington's Pharmaceutical Sciences by E.W. Martin. See also Wang, Y.J. and Hanson, M.A. "parenteral Formulations of Proteins and Peptides: Stability and Stabilizers," Journal of Parenteral Science and Technology, Technical Report No. 10, Supp. 42:25 (1988).

Compounds useful in the invention can be provided as parenteral compositions for injection or infusion. They can, for example, be suspended in an inert oil, suitably a vegetable oil such as sesame, peanut, olive oil, or other acceptable carrier. Preferably, they are suspended in an aqueous carrier, for example, in an isotonic buffer solution at a pH of about 4.0 to about 7.4. These compositions may be sterilized by conventional sterilization techniques, or may be sterile filtered. The compositions may contain pharmaceutically acceptable auxiliary substances as required to approximate physiological conditions, such as pH buffering agents. Useful buffers include for example, sodium acetate/acetic acid buffers. A form of repository or "depot" slow release preparation may be used so that therapeutically effective amounts of the preparation are delivered into the bloodstream over many hours or days following transdermal injection or delivery.

The desired isotonicity may be accomplished using sodium chloride or other pharmaceutically acceptable agents such as dextrose, boric acid, sodium tartrate, propylene glycol, polyols (such as mannitol and sorbitol), or other inorganic or organic solutes. Sodium chloride is preferred particularly for buffers containing sodium ions.

The claimed compounds can also be formulated as pharmaceutically acceptable salts (*e.g*., acid addition salts) and/or complexes thereof. Pharmaceutically acceptable salts are non-toxic salts at the concentration at which they are administered. The preparation of such salts can facilitate the pharmacological use by altering the physical-chemical characteristics of the composition without preventing the composition from exerting its physiological effect. Examples of useful alterations in physical properties include lowering the melting point to facilitate transmucosal administration and increasing the solubility to facilitate the administration of higher concentrations of the drug.

Pharmaceutically acceptable salts include acid addition salts such as those containing sulfate, hydrochloride, phosphate, sulfamate, acetate, citrate, lactate, tartrate, methanesulfonate, ethanesulfonate, benzenesulfonate, *p-*toluenesulfonate, cyclohexylsulfamate and quinate. Pharmaceutically acceptable salts can be obtained from acids such as hydrochloric acid, sulfuric acid, phosphoric acid, sulfamic acid, acetic acid, citric acid, lactic acid, tartaric acid, malonic acid, methanesu-lfonic acid, ethanesulfonic acid, benzenesulfonic acid, *p*-toluenesulfonic acid, cyclohexylsulfamic acid, and quinic acid. Such salts may be prepared by, for example, reacting the free acid or base forms of the product with one or more equivalents of the appropriate base or acid in a solvent or medium in which the salt is insoluble, or in a solvent such as water which is then removed in vacuo or by freeze-drying or by exchanging the ions of an existing salt for another ion on a suitable ion exchange resin.

Carriers or excipients can also be used to facilitate administration of the compound. Examples of carriers and excipients include calcium carbonate, calcium phosphate, various sugars such as lactose, glucose, or sucrose, or types of starch, cellulose derivatives, gelatin, vegetable oils, polyethylene glycols and physiologically compatible solvents. The compositions or pharmaceutical composition can be administered by different routes including intravenously, intraperitoneal, subcutaneous, and intramuscular, orally, topically, or transmucosally.

If desired, solutions of the above compositions may be thickened with a thickening agent such as methyl cellulose. They may be prepared in emulsified form, either water in oil or oil in water. Any of a wide variety of pharmaceutically acceptable emulsifying agents may be employed including, for example, acacia powder, a non-ionic surfactant (such as a Tween), or an ionic surfactant (such as alkali polyether alcohol sulfates or sulfonates, *e.g*., a Triton).

Compositions useful in the invention are prepared by mixing the ingredients following generally accepted procedures. For example, the selected components may be simply mixed in a blender or other standard device to produce a concentrated mixture which may then be adjusted to the final concentration and viscosity by the addition of water or thickening agent and possibly a buffer to control pH or an additional solute to control tonicity.

For use by the physician, the compounds will be provided in dosage unit form containing an amount of an exendin agonist, with or without another anti-emptying agent. Therapeutically effective amounts of an exendin agonist for use in the control of gastric emptying and in conditions in which gastric emptying is beneficially slowed or regulated are those that decrease post-prandial blood glucose levels, preferably to no more than about 8 or 9 mM or such that blood glucose levels are reduced as desired. In diabetic or glucose intolerant individuals, plasma glucose levels are higher than in normal individuals. In such individuals, beneficial reduction or "smoothing" of post-prandial blood glucose levels, may be obtained. As will be recognized by those in the field, an effective amount of therapeutic agent will vary with many factors including the age and weight of the patient, the patient's physical condition, the blood sugar level or level of inhibition of gastric emptying to be obtained, and other factors.

Such pharmaceutical compositions are useful in causing gastric hypomotility in a subject and may be used as well in other disorders where gastric motility is beneficially reduced.

The effective daily anti-emptying dose of the compounds will typically be in the range of 0.01 or 0.03 to about 5 mg/day, preferably about 0.01 or 0.5 to 2 mg/day and more preferably about 0.01 or 0.1 to 1 mg/day, for a 70 kg patient, administered in a single or divided doses. The exact dose to be administered is determined by the attending clinician and is dependent upon where the particular compound lies within the above quoted range, as well as upon the age, weight and condition of the individual. Administration should begin at the first sign of symptoms or shortly after diagnosis of diabetes mellitus. Administration may be by injection, preferably subcutaneous or intramuscular. Orally active compounds may be taken orally, however dosages should be increased 5-10 fold.

Generally, in treating or preventing elevated, inappropriate, or undesired post-prandial blood glucose levels, the compounds of this invention may be administered to patients in need of such treatment in a dosage ranges similar to those given above, however, the compounds are administered more frequently, for example, one, two, or three times a day.

The optimal formulation and mode of administration of compounds of the present application to a patient depend on factors known in the art such as the particular disease or disorder, the desired effect, and the type of patient. While the compounds will typically be used to treat human patients, they may also be used to treat similar or identical diseases in other vertebrates such as other primates, farm animals such as swine, cattle and poultry, and sports animals and pets such as horses, dogs and cats.

To assist in understanding the present invention the following Examples are included which describe the results of a series of experiments. The experiments relating to this invention should not, of course, be construed as specifically limiting the invention and such variations of the invention, now known or later developed, which would be within the purview of one skilled in the art are considered to fall within the scope of the invention as described herein and hereinafter claimed.

### EXAMPLE 1 - PREPARATION OF EXENDIN-3

### His Ser Asp Gly Thr Phe Thr Ser Asp Leu Ser Lys Gln Met Glu Glu Glu Ala Val Arg Leu Phe Ile Glu Trp Leu Lys Asn Gly Gly Pro Ser Ser Gly Ala Pro Pro Pro Ser-NH₂ [SEQ. ID. NO. 1]

The above amidated peptide was assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.). In general, single-coupling cycles were used throughout the synthesis and Fast Moc (HBTU activation) chemistry was employed. Deprotection (Fmoc group removal)of the growing peptide chain was achieved using piperidine. Final deprotection of the completed peptide resin was achieved using a mixture of triethylsilane (0.2 mL), ethanedithiol (0.2 mL), anisole (0.2 mL), water (0.2 mL) and trifluoroacetic acid (15 mL) according to standard methods (Introduction to Cleavage Techniques, Applied Biosystems, Inc.) The peptide was precipitated in ether/water (50 mL) and centrifuged. The precipitate was reconstituted in glacial acetic acid and lyophilized. The lyophilized peptide was dissolved in water). Crude purity was about 75%.

Used in purification steps and analysis were Solvent A (0.1% TFA in water) and Solvent B (0.1% TFA in ACN).

The solution containing peptide was applied to a preparative C-18 column and purified (10% to 40% Solvent B in Solvent A over 40 minutes). Purity of fractions was determined isocratically using a C-18 analytical column. Pure fractions were pooled furnishing the above-identified peptide. Analytical RP-HPLC (gradient 30% to 60% Solvent B in Solvent A over 30 minutes) of the lyophilized peptide gave product peptide having an observed retention time of 19.2 minutes.

### EXAMPLE 2 - PREPARATION OF EXENDIN-4

His Gly Glu Gly Thr Phe Thr Ser Asp Leu Ser Lys Gln Met Glu
Glu Glu Ala Val Arg Leu Phe Ile Glu Trp Leu Lys Asn Gly Gly
Pro Ser Ser Gly Ala Pro Pro Pro Ser-NH2 [SEQ. ID. NO. 2]

The above amidated peptide was assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Exendin-3 as describe in Example 1. Used in analysis were Solvent A (0.1% TFA in water) and Solvent B (0.1% TFA in ACN). Analytical RP-HPLC (gradient 36% to 46% Solvent B in Solvent A over 30 minutes) of the lyophilized peptide gave product peptide having an observed retention time of 14.9 minutes. Electrospray Mass Spectrometry (M): calculated 4186.6; found 4186.0 to 4186.8 (four lots).

### EXAMPLE 3: CLEARANCE BY THE KIDNEY

The kidney can play a major role in the elimination of some molecules (drugs, peptides, proteins). For some molecules, this process begins when the kidney filters the blood at the glomerulus to produce the ultrafiltrate described below. The glomerular filter discriminates not only on the basis of molecular weight but also by acting as a negatively charged selective barrier, promoting retention of anionic compounds. The free fraction of molecules in the plasma (not protein bound) with a molecular weight less than 5kD and an effective radii less than 15 Å are easily filtered. For larger molecular weight molecules they are filtered on a more restrictive and limited basis, principally by molecular size, structure and net charge. The cutoff point for glomerular filtration lies between albumin (67kD) which is retained and hemoglobin (68kD) which is filtered. Albumin, with an effective radius of about 36 Å is filtered less than 1% at the glomerulus.

Once in the glomerulus a molecule travels to the proximal tubule where it is either reabsorbed or it passes on through the loop of Henle to the distal tubule where collecting ducts drain the filtrate into the bladder. Filtered proteins and peptides are typically cleaved by brush border enzymes in the proximal tubule, from where they are efficiently retrieved by sodium/amino cotransporters (scavenging pumps). Otherwise, molecules which are polar, ionized and of large molecular weight will not be reabsorbed. Throughout this process metabolizing enzymes in the renal cortex (proximal tubules) may also degrade the molecule into more polar molecules, thereby increasing the probability for excretion into the urine. Many peptide hormones (for example, amylin, calcitonins, and GLP-1) are degraded by passage through the renal circulation, presumably by vascular ectoenzymes accessible to the plasma, independently of the process of glomerular filtration. In those examples, rates of peptide clearance from the plasma are similar to the rate of renal plasma flow, which is ∼3-fold greater than the rate of glomerular filtration.

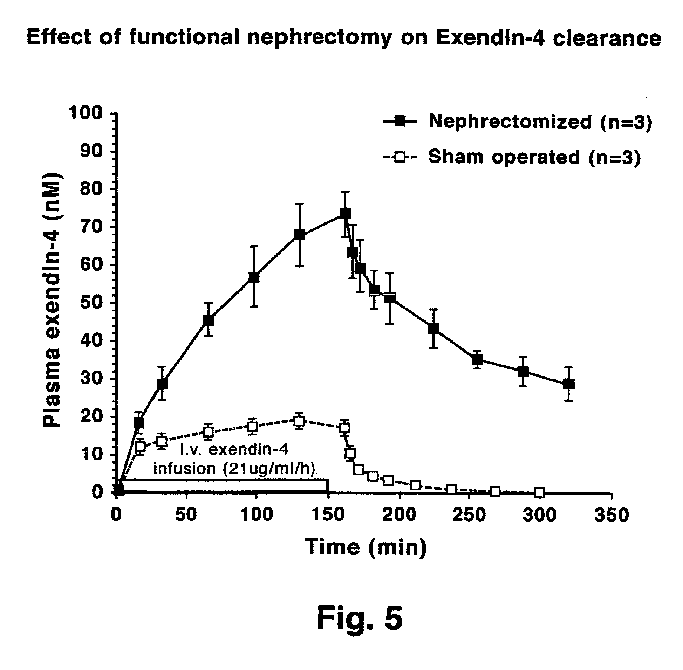

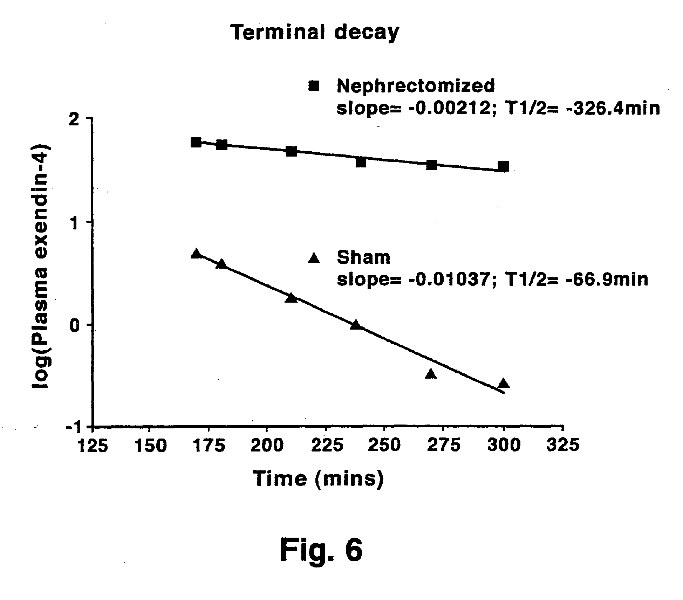

To test whether renal filtration could be the principal mode of exendin elimination, studies were performed in overnight fasted nephrectomized male rats infused with exendin-4 at a constant rate. Steady-state plasma levels of exendin-4 were greatly increased in nephrectomized rats compared to rats with their kidneys intact. This data indicated that the kidney was responsible for at least 80% of the clearance of exendin-4 (see Figures 5 and 6). Exendin-4 clearance rates in intact rats were similar to glomerular filtration rates expected in those rats (4.2 mL/min). Taken together these results indicate that very little metabolism seems to occur systemically and that most of the clearance of exendin-4 is through the kidney via filtration (but not by renal intravascular proteolysis). The low amounts of immunoreactive full-length exendin-4 in the urine are consistent with it being cleaved by brush border enzymes in the proximal tubule after filtration. These results are also consistent with the fact that studies performed to identify plasma circulating metabolites of exendin-4 yielded very little evidence of proteolytic degradation; following large intravenous doses in animals, HPLC analysis of plasma showed only the presence of intact exendin, and negligible appearance of "daughter" peaks indicative of the buildup of degradation products. This is in contrast to other peptides studied (for example amylin and GLP-1), where the disappearance of the "parent" HPLC peak was associated with the appearance of "daughter" HPLC peaks, subsequently identified as subpeptide degradants.

### EXAMPLE 4: PEG MODIFIED EXENDIN-4

Different spectra of biological activities of exendin-4 may be selected by putting a PEG group at appropriate positions. Loss or alteration of bioactivity has been reported for PEGylated proteins which may be due to the presence of the PEG chains themselves, the particular site occupied by the PEG chain, or the coupling conditions having an adverse effect on the protein.

Primary considerations for PEG modification in terms of filtration at the kidney of exendin and exendin agonists are size and charge. Unmodified, exendin-4 has a molecular weight of approximately 4.2 kD and is anionic in nature with an overall net charge of approximately -2 at physiological pH. One to ten, preferably one, two or three PEG constituents may be covalently linked to exendin-4 or an analog of exendin-4, for example, with one PEG constituent being preferred. The size of each independent PEG constituent can vary from a molecular weight of 500 to 20,000, preferably between 5,000 and 12,000.

Many of the methods for covalent attachment of PEG involve the epsilon-amino group on lysine. Exendin-4 has two lysines that could be modified by attachment of PEG(see compounds 201 and 202, below). In addition, the epsilon-amino groups at these positions may be masked, thereby increasing the anionic nature of the peptide.

| | |
|---|---|
| (201) | HGEGTFTSDLSK(PEG)QMEEEAVRLFIEWLKNGGPSSGAPPPS-NH₂ |
| (202) | HGEGTFTSDLSKQMEEEAVRLFIEWLK(PEG)NGGPSSGAPPPS-NH2 |

Other positions that may be modified by substitution of a Lys-PEG or equivalent, for example, are:

| | |
|---|---|
| (203) | HK(PEG)EGTFTSDLSKQMEEEAVRLFIEWLKNGGPSSGAPPPS-NH₂ |
| (204) | HGEGK(PEG)FTSDLSKQMEEEAVRLFIEWLKNGGPSSGAPPPS-NH₂ |
| (205) | HGEGTFTK(PEG)DLSKQMEEEAVRLFIEWLKNGGPSSGAPPPS-NH₂ |
| (206) | HGEGTFTSDK(PEG)SKQMEEEAVRLFIEWLKNGGPSSGAPPPS-NH_{Z} |
| (207) | HGEGTFTSDLK(PEG)KQMEEEAVRLFIEWLKNGGPSSGAPPPS-NH₂ |
| (208) | HGEGTFTSDLSKK(PEG)MEEEAVRLFIEWLKNGGPSSGAPPPS-NH₂ |
| (209)* | HGEGTFTSDLSKQMEK(PEG)EAVRLFIEWLKNGGPSSGAPPPS-NH₂ |
| (210)* | HGEGTFTSDLSKQMEEK(PEG)AVRLFIEWLKNGGPSSGAPPPS-NH₂ |
| (211) | HGEGTFTSDLSKQMEEEAK(PEG)RLFIEWLKNGGPSSGAPPPS-NH₂ |
| (212) | HGEGTFTSDLSKQMEEEAVRK(PEG)FIEWLKNGGPSSGAPPPS-NH₂ |
| (213)* | HGEGTFTSDLSKQMEEEAVRLFIK(PEG)WLKNGGPSSGAPPPS-NH₂ |
| (214) | HGEGTFTSDLSKQMEEEAVRLFIEK(PEG)LKNGGPSSGAPPPS-NH₂ |
| (215) | HGEGTFTSDLSKQMEEEAVRLFIEWLKK(PEG)GGPSSGAPPPS-NH₂ |

The three molecules marked with an asterisk above contain a PEGylated Lys residue substituted for a glutamic acid at the specified location. Those in the art will appreciate that non-K(PEG) substituted molecules at these positions can instead be modified by conjugation of a PEG moiety to the glutamic side chain carboxyl group, which modification is referred to herein as E(PEG).

Other analogs in which Lys-PEG can be substituted include:
(216) HGEGTFTSDLSKQMEEEAVRLFIEWLKNK(PEG)GPSSGAPPPS-NH₂
(217) HGEGTFTSDLSKQMEEEAVRLFIEWLKNGK(PEG)PSSGAPPPS-NH₂

Various molecules, including K(PEG) modified and arginine substituted exendins, used in Examples 5-10 are shown in Table I, below.

**Table I**

| | |
|---|---|
| exendin- | HGEGTFTSDLSKQMEEEAVRLFIEWLKNGGPSSGAPPPS-NH₂ |
| 4 | |
| (218) | (CH3)-COHGEGTFTSDLSKQMEEEAVRLFIEWLKNGGPSSGAPPPS-NH₂ |
| (219) | (CH3)-CH2HGEGTFTSDLSKQMEEEAVRLFIEWLKNGGPSSGAPPPS-NH₂ |
| (220) | HGEGTFTSDLS**R**QMEEEAVRLFIEWLK(PEG)NGGPSSGAPPPS-NH₂ |
| (221) | HGEGTFTSDLSK(PEG)QMEEEAVRLFIEWL**R**NGGPSSGAPPPS-NH₂ |
| (222) | HGEGTFTSDLS**R**QMEEEAVRLFIEWL**R**NGGPSSGAPPPS-NH₂ |
| (223) | HGEGTFTSDLS**R**QMEEEAVRLFIEWL**R**NGGPSSGAPPPK(PEG)-NH₂ |
| (224) | HGEGTFTSDLS**R**QMEEEAVRLFIEWL**R**NGK(PEG)PSSGAPPPS-NH₂ |
| (225) | HGEGTFTSDLS**R**QMEEEAVRLFIEWLK(PEG)NGGPSSGAPPPS-NH₂ |
| (226) | HGEGTFTSDLSK(PEG)QMEEEAVRLFIEWL**R**NGGPSSGAPPPS-NH₂ |
| (227) | (PEG)COHGEGTFTSDLS**R**QMEEEAVRLFIEWL**R**NGGPSSGA-PPPS-NH₂ |
| (228) | (PEG)CH2HGEGTFTSDLS**R**QMEEEAVRLFIEWL**R**NGGPSSGAPPPS-NH₂ |
| (229) | HGEGTFTSDLS**R**QMEEEAVRLFIEWL**R**NGGPSSGAPPPK(PEG)-NH₂ |
| (230) | HGEGTFTSDLS**R**QMEEEAVRLFIEWL**R**NGK(PEG)PSSGAPPPS-NH₂ |

The various PEG modified exendins used in Examples 5-10, below, are provided in Table I, with the corresponding results being provided in Table II (see the end of Example 9). GLP-1[7-36]NH₂ (GLP-1) was purchased from Bachem (Torrance, CA). All other peptides were prepared using synthesis methods such as those described herein. All chemicals were of the highest commercial grade. The cAMP SPA immunoassay was purchased from Amersham. The radioligands were purchased from New England Nuclear (Boston, MA). RINm5f cells (American Type Tissue Collection, Rockville, MD) were grown in DME/F12 medium containing 10% fetal bovine serum and 2mM L-glutamine. Cells were grown at 37°C and 5% CO₂/95% humidified air and medium was replaced every 2 to 3 days. Cells were grown to confluence then harvested and homogenized using on a Polytron homogenizer. Cell homogenates were stored frozen at -70°C until used.

### EXAMPLE 5 - GLP-1 RECEPTOR BINDING STUDIES

Receptor binding can be assessed by measuring displacement of [¹²⁵I]GLP-1 or [¹²⁵I]exendin(9-39) from RINm5f membranes. Assay buffer contained 5 µg/ml bestatin, 1 µg/ml phosphoramidon, 1 mg/ml bovine serum albumin (fraction V), 1 mg/ml bacitracin, and 1 mM MgCl₂ in 20 mM HEPES, pH 7.4. To measure binding, 30 µg membrane protein (Bradford protein assay) is resuspended in 200 µl assay buffer and incubated with 60 pM [¹²⁵I]GLP-1 or [¹²⁵I]exendin(9-39) and unlabeled peptides for 120 minutes at 23□C in 96 well plates (Nagle Nunc, Rochester, NY). Incubations are terminated by rapid filtration with cold phosphate buffered saline, pH 7.4, through polyethyleneimine-treated GF/B glass fiber filters (Wallac Inc., Gaithersburg, MD) using a Tomtec Mach II plate harvester (Wallac Inc., Gaithersburg, MD). Filters are dried, combined with scintillant, and radioactivity determined in a Betaplate liquid scintillant counter (Wallac Inc.).

Peptide samples are run in the assay as duplicate points at 6 dilutions over a concentration range of 10⁻⁶M to 10⁻¹²M to generate response curves. The biological activity of a sample can be expressed as an IC₅₀ value, calculated from the raw data using an iterative curve-fitting program using a 4-parameter logistic equation (Prizm, GraphPAD Software).

### EXAMPLE 6 - CYCLASE ACTIVATION STUDY

Assay buffer contained 10 µM GTP, 0.75 mM ATP, 2.5 mM MgCl₂, 0.5mM phosphocreatine, 12.5 U/ml creatine kinase, 0.4 mg/ml aprotinin, 1 µM IBMX in 50 mM HEPES, pH 7.4. Membranes and peptides was combined in 100 ml of assay buffer in 96 well filter-bottom plates (Millipore Corp., Bedford, MA). After 20 minutes incubation at 37°C, the assay was terminated by transfer of supernatant by filtration into a fresh 96 well plate using a Millipore vacuum manifold. Supernatant cAMP contents were quantitated by SPA immunoassay. Peptide samples were run in the assay as triplicate points at 7 dilutions over a concentration range of 10⁻⁶M to 10⁻¹²M to generate response curves. The biological activity of a particular sample was expressed as an EC₅₀ value calculated as described above.

### EXAMPLE 7 - DETERMINATION OF BLOOD GLUCOSE LEVELS IN DB/DB MICE

C57BLKS/J-m-db mice at least 3 months of age are utilized for the study. The mice can be obtained from The Jackson Laboratory and allowed to acclimate for at least one week before use. Mice can be housed in groups of ten at 22°C ± 1°C with a 12:12 light:dark cycle, with lights on at 6 a.m. All animals can be deprived of food for 2 hours before taking baseline blood samples. Approximately 70 µl of blood is drawn from each mouse via eye puncture, after a light anesthesia with metophane. After collecting baseline blood samples, to measure plasma glucose concentrations, all animals receive subcutaneous injections of either vehicle (10.9% NaCl), exendin-4 or test compound (1 µg) in vehicle. Blood samples were drawn again, using the same procedure, after exactly one hour from the injections, and plasma glucose concentrations were measured. For each animal, the % change in plasma value, from baseline value, was calculated.

### EXAMPLE 8 - DOSE RESPONSE DETERMINATION OF BLOOD GLUCOSE LEVELS IN DB/DB MICE

C57BLKS/J-m-db/db mice, at least 3 months of age, were utilized. The mice were obtained from The Jackson Laboratory and allowed to acclimate for at least one week before use. Mice were housed in groups of ten at 22°C ± 1°C with a 12:12 light:dark cycle, with lights on at 6 a.m. All animals were deprived of food for 2 hours before taking baseline blood samples. Approximately 70 µl of blood was drawn from each mouse via eye puncture, after a light anesthesia with metophane. After collecting baseline blood samples, to measure plasma glucose concentrations, all animals receive subcutaneous injections of either vehicle, exendin-4 or test compound. Blood samples were drawn again, using the same procedure, after exactly one hour from the injections, and plasma glucose concentrations were measured. For each animal, the % change in plasma value, from baseline value, was calculated and a dose dependent relationship was evaluated using Graphpad Prizm™ software.

### EXAMPLE 9 - GASTRIC EMPTYING

A gastric emptying study may also be carried out to examine the effects of exendin-4 and/or an exendin agonist compound on gastric emptying in rats. Such experiments typically follow a modification of the method of Scarpignato, et al., Arch. Int. Pharmacodyn. Ther. 246:286-94, 1980. Male Harlan Sprague Dawley (HSD) rats are used. All animals are housed at 22.7 ± 0.8°C in a 12:12 hour light:dark cycle (experiments being performed during the light cycle) and were fed and watered *ad libitum* (Diet LM-485, Teklad, Madison, WI). The determination of gastric emptying by the method described below can be performed after a fast of ∼20 hours to ensure that the stomach contained no chyme that would interfere with spectrophotometric absorbance measurements.

Conscious rats receive by gavage 1.5ml of an acaloric gel containing 1.5% methyl cellulose (M-0262, Sigma Chemical Co, St Louis, MO) and 0.05% phenol red indicator. Twenty minutes after gavage, rats are anesthetized using 5% halothane, the stomach is exposed and clamped at the pyloric and lower esophageal sphincters using artery forceps, removed and opened into an alkaline solution made up to a fixed volume. Stomach content is derived from the intensity of the phenol red in the alkaline solution, measured by absorbance at a wavelength of 560 nm. In separate experiments on several other rats, the stomach and small intestine can be both excised and opened into an alkaline solution. The quantity of phenol red that could be recovered from the upper gastrointestinal tract within 20 minutes of gavage can then be determined. Dye which appears to bind irrecoverably to the gut luminal surface accounts for the balance. To account for a maximal dye recovery of less than 100%, the percentage of stomach contents remaining after 20 min. are expressed as a fraction of the gastric contents recovered from control rats sacrificed immediately after gavage in the same experiment. Percent gastric contents remaining = (absorbance at 20 min)/(absorbance at 0 mm) x 100.

### EXAMPLE 10 - Test Compound Injections Reduced Food Intake in Normal Mice

All mice (NIH:Swiss mice) were housed in a stable environment of 22 (± 2)° C, 60 (±10) % humidity and a 12:12 light:dark cycle; with lights on at 0600. Mice were housed in groups of four in standard cages with *ad libitum* access to food (Teklad: LM 485; Madison, WI) and water except as noted, for at least two weeks before the experiments.

All experiments were conducted between the hours of 0700 and 0900. The mice were food deprived (food removed at 1600 hr from all animals on day prior to experiment) and thereafter individually housed. All mice received an intraperitoneal injection (5 µl/kg) of either saline or test compound at doses of 0.1, 1.0, 10, and 100 µg/kg, and were immediately presented with a pre-weighed food pellet (Teklad LM 485). The food pellet was weighed at 30-minute, 1-hr, 2-hr and 6-hr intervals to determine the amount of food eaten. The ED₅₀ for inhibition of food intake over 30 min was determined for several test compounds, and the results appear in Table II, below.

**Table II**

| | **GLP-1 Cyclase EC50 nM** | **Appetite Suppression ED50 µg/kg** |
|---|---|---|
| exendin4 | 0.27 | 0.21 |
| 218 | >1000 | 1.80 |
| 219 | 1.11 | 0.08 |
| 220 | 0.8 | 0.12 |
| 221 | 0.69 | 6.70 |
| 222 | 2.70 | weak |
| 223 | 0.46 | 2.40 |
| 224 | 3.22 | weak |
| 225 | 23 | weak |
| 226 | 102 | 2.40 |
| 227 | 149 | NA |
| 228 | 458 | NA |
| 229 | 60.4 | 14.50 |
| 230 | 157 | NA |

One skilled in the art would readily appreciate that the present invention is well adapted to carry out the objects and obtain the ends and advantages mentioned, as well as those inherent therein. The molecular complexes and the methods, procedures, treatments, molecules, specific compounds described herein are presently representative of preferred embodiments are exemplary and are not intended as limitations on the scope of the invention. Changes therein and other uses will occur to those skilled in the art which are encompassed within the spirit of the invention are defined by the scope of the claims.

It will be readily apparent to one skilled in the art that varying substitutions and modifications may be made to the invention disclosed herein without departing from the scope and spirit of the invention.

All patents and publications mentioned in the specification are indicative of the levels of those skilled in the art to which the invention pertains. All patents and publications are herein incorporated by reference in its entirity to the same extent as if each individual publication was specifically and individually indicated to be so incorporated by reference.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

In addition, where features or aspects of the invention are described in terms of Markush groups, those skilled in the art will recognize that the invention is also thereby described in terms of any individual member or subgroup of members of the Markush group. For example, if X is described as selected from the group consisting of bromine, chlorine, and iodine, claims for X being bromine and claims for X being bromine and chlorine are fully described.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims.

The claims of the parent application are reproduced below. These clauses define preferable combinations of features. The applicant reserves the right to pursue protection for these combinations of features, and/or any other subject-matter contained in the parent application as filed, either in the present divisional application or in a further application divided from the present divisional application. The claims of the parent application are not the claims of the current application which are contained in a separate section headed "claims".
1. A modified exendin or exendin agonist comprising an exendin or exendin agonist linked to one or more polyethylene glycol polymers.
2. The modified exendin or exendin agonist of claim 1, wherein said exendin or exendin agonist is exendin-4.
3. The modified exendin or exendin agonist of claim 1, wherein said exendin or exendin agonist is linked to one polyethylene glycol polymer.
4. The modified exendin or exendin agonist of claim 1, wherein said exendin or exendin agonist is linked to two polyethylene glycol polymers.
5. The modified exendin or exendin agonist of claim 1, wherein said exendin or exendin agonist is linked to three polyethylene glycol polymers.
6. The modified exendin or exendin agonist of any one of claims 1-5, wherein said one or more polyethylene glycol polymers each have molecular weights between 500 and 20,000.
7. The modified exendin or exendin agonist of any one of claims 1-5, wherein said exendin or exendin agonist is linked to said one or more polyethylene glycol polymers through an epsilon amino group on a lysine amino acid of said exendin or exendin agonist.
8. The modified exendin or exendin agonist of claim 1, wherein said modified exendin or exendin agonist is selected from the group of compounds consisting of compounds 201-230.
9. The modified exendin or exendin agonist of claim 1, wherein said modified exendin or exendin agonist is selected from the group of compounds consisting of compounds 209, 210 and 213.
10. The modified exendin or exendin agonist of claim 1, wherein said modified exendin or exendin agonist is selected from the group of compounds consisting of compounds 201 and 202.
11. The modified exendin or exendin agonist of claim 1, wherein said modified exendin or exendin agonist is selected from the group of compounds consisting of compounds 216 and 217.
12. The modified exendin or exendin agonist of claim 1, wherein said one or more polyethylene glycol polymers are linked to an amino, carboxyl, or thio group of said exendin or exendin agonist.
13. The modified exendin or exendin agonist of claim 1, wherein said one or more polyethylene glycol polymers are linked to the N or C termini, or the N and C termini of side chains of one or more amino acids of said exendin or exendin agonist, wherein said amino acids are selected from the group consisting of lysine, aspartic acid, glutamic acid, and cysteine.
14. The modified exendin or exendin agonist of claim 1, wherein said one or more polyethylene glycol polymers are linked to said exendin or exendin agonist with one or more amino acid side chain moities with amine or carboxylic groups, or amine and carboxylic groups.
15. A method of making a modified exendin or exendin agonist of claim 1, comprising linking said one or more polyethylene glycol polymer to said exendin or exendin agonist.
16. The method of claim 15, wherein said linking is performed by solid-phase synthesis.
17. A method of treating a disease benefited by administration of an exendin or exendin agonist, comprising the step of providing a modified exendin or exendin agonist of claim 1 to a patient having said disease and thereby treating said disease.
18. The method of claim 17, wherein said disease is selected from the group consisting of postprandial dumping syndrome, postprandial hyperglycemia, impaired glucose tolerance, a condition or disorder which can be alleviated by suppressing glucagon secretion, modulating triglyceride levels, reducing food intake, obesity, an eating disorder, insulin-resistance syndrome, diabetes mellitus, a hyperglycemic condition, and a hypoglycemic condition.
19. A pharmaceutical composition comprising a modified exendin or exendin agonist of claim 1 and a pharmaceutically acceptable carrier.
20. A kit comprising a modified exendin or exendin agonist of claim 1 and instructions or packaging for use.
21. A method of beneficially regulating gastro-intestinal motility in a subject comprising administering to said subject a therapeutically effective amount of a modified exendin or exendin agonist of claim 1.
22. A method of treatment for ingestion of a toxin comprising: (a) administering an amount of a modified exendin or exendin agonist of claim 1 effective to prevent or reduce the passage of stomach contents to the intestines; and (b) aspirating the contents of the stomach.
23. A method for reducing the appetite or weight, or lowering plasma lipids, of a subject comprising administering to said subject a therapeutically effective amount of a modified exendin or exendin agonist of claim 1.
24. A method for modulating triglyceride levels in a subject, comprising administering to said subject a therapeutically effective amount of a modified exendin or exendin agonist of claim 1.
25. A method for suppressing glucagon secretion in a subject, comprising administering to said subject a therapeutically effective amount of a modified exendin or exendin agonist of claim 1.
26. A method for treating diabetes mellitus in a subject, comprising administering to said subject a therapeutically effective amount of a modified exendin or exendin agonist of claim 1.
27. A method according to claim 26 wherein the diabetes mellitus is selected from the group consisting of Type 1 diabetes, Type 2 diabetes, and gestational diabetes.
28. A pharmaceutical composition for use in the treatment of conditions or disorders associated with hypernutrition, or in reducing the appetite or weight of a subject, or in suppressing glucagon secretion, or in modulating triglceride levels, or for use in lowering the plasma lipid level of a subject, comprising a therapeutically effective amount of a modified exendin or exendin agonist of claim 1 in association with a pharmaceutically acceptable carrier.
29. A modified exendin or exendin agonist comprising an exendin or exendin agonist linked to one or more molecular weight increasing compounds.
30. A modified exendin or exendin agonist according to claim 29 wherein at least one of the molecular weight increasing compounds is selected from the group consisting of a polyethylene glycol polymer, albumin, a polyamino acid, gelatin, succinyl-gelatin, poly((hydroxypropyl)methacrylamide), a fatty acid, a olysaccaride, a lipid amino acid, and dextran.
31. The use of a modified exendin or exendin agonist according to claim 30 for the preparation of a medicament.
32. A method of treatment of a subject comprising administering to said subject in need thereof a modified exendin or exendin agonist according to claim 30 in a pharmaceutically acceptable character.
33. A modified exendin or exendin agonist according to claim 29 which is a modified exendin-4.
34. The use according to claim 31 wherein said modified exendin or exendin agonist is a modified exendin-4.
35. The method according to claim 32 which said modified exendin or exendin agonist is a modified exendin-4.

## Claims

1. A modified exendin or exendin agonist comprising an exendin or exendin agonist linked to one or more molecular weight increasing compounds.

2. A modified exendin or exendin agonist according to claim 1 wherein at least one of the molecular weight increasing compounds is selected from the group consisting of a polyamino acid, albumin, a polyethylene glycol polymer, gelatin, succinyl-gelatin, poly((hydroxypropyl)methacrylamide), a fatty acid, a olysaccharide, a lipid amino acid, and dextran.

3. A modified exendin or exendin agonist according to claim 2 wherein at least one of the molecular weight increasing compounds is a polyamino acid.

4. A modified exendin or exendin agonist according to claim 3 wherein the polyamino acid is poly-lysine, poly-glutamic acid and poly-aspartic acid.

5. A modified exendin or exendin agonist according to claim 4 wherein the polyamino acid is poly-lysine.

6. A modified exendin or exendin agonist according to claim 2 wherein at least one of the molecular weight increasing compounds is albumin.

7. A modified exendin or exendin agonist according to claim 2 wherein at least one of the molecular weight increasing compounds is a polyethylene glycol polymer.

8. A modified exendin or exendin agonist according to any one of claims 1 to 7 wherein the one or more molecular weight increasing compounds are linked to the C-terminal of the exendin or exendin agonist.

9. A modified exendin or exendin agonist according to any one of claims 1 to 8 which is a modified exendin-4.

10. A modified exendin or exendin agonist according to any one of claims 1 to 9 for use in the treatment of postprandial dumping syndrome, postprandial hyperglycemia, impaired glucose tolerance, a condition or disorder which can be alleviated by reducing food intake, obesity, an eating disorder, insulin-resistance syndrome, diabetes mellitus or a hyperglycemic condition.

11. A modified exendin or exendin agonist according to claim 10 for use in the treatment of diabetes, postprandial hyperglycemia or impaired glucose tolerance.

12. A modified exendin or exendin agonist according to any one of claims 1 to 9 for use in a method of treatment of a subject comprising administering to said subject in need thereof the modified exendin or exendin agonist in a pharmaceutically acceptable character.

13. A pharmaceutical composition comprising a modified exendin or exendin agonist according to any one of claims 1 to 9 and a pharmaceutically acceptable carrier.

14. Use of a modified exendin or exendin agonist according to any one of claims 1 to 9 for the preparation of a medicament.
